# EUROPEAN PATENT APPLICATION

(11) **EP 4 847 489 A1**
(43) Date of publication of application: **02.12.2026**
(21) Application number: 24926926.7
(22) Date of filing: 31.12.2024
(51) Int. Cl.: C07D 233/96, C07D 401/06, C07D 401/14, C07D 405/06, C07D 409/06, A61K 31/4709, A61K 31/4439, A61K 31/4166, A61K 31/4178, A61P 35/00, A61P 35/02, A61P 3/10, A61P 37/02

(54) **IMIDAZOLIDINEDIONE HDAC INHIBITOR, AND PREPARATION METHOD THEREFOR AND USE THEREOF**

(30) Priority: 28.02.2024 CN 202410218988
(71) Applicant: Nanjing Hongshun Pharmaceutical Technology Co., Ltd, Nanjing, Jiangsu 210000 (CN); China Pharmaceutical University, Nanjing, Jiangsu 210000 (CN)
(72) Inventor: ZHOU, Qingfa, Nanjing, Jiangsu 210000 (CN); YAO, Shengfa, Nanjing, Jiangsu 210000 (CN); WANG, Enyuan, Nanjing, Jiangsu 210000 (CN); ZHANG, Yufei, Nanjing, Jiangsu 210000 (CN); JIA, Shuolei, Nanjing, Jiangsu 210000 (CN)
(74) Representative: Bayramoglu et al.
(86) International application number: PCT/CN2024/144071
(87) International publication number: WO 2025/180068

(57) **Abstract**

The present invention relates to an imidazolidinedione-based HDAC inhibitor, a method for preparing the same and use thereof, and belongs to the technical field of medicines. The imidazolidinedione-based HDAC inhibitor provided by the present invention is a substituted imidazolidinedione compound of Formula V, or a stereisomer, hydrate or pharmaceutically acceptable salt thereof. The inhibitor has efficient and excellent HDAC inhibitory activity, as well as good in vitro anti-tumor activity, providing a new approach for the application of an HDAC inhibitor in tumors or the treatment of diseases related to uncontrolled histone deacetylase activity.

## Description

### TECHNICAL FIELD

The present invention relates to an imidazolidinedione-based HDAC inhibitor, a method for preparing the same and use thereof, and belongs to the technical field of medicines.

### BACKGROUND TECHNOLOGY

Traditional therapies for malignant tumors mainly include surgery, chemotherapy and radiotherapy. Although the surgery is an effective method, it is not ideal for prolonging the life of a patient if other adjuvant therapies are not further carried out. The chemotherapy may lead to drug resistance and cause side effects, typically including bone marrow suppression, impaired intestinal function, etc. The radiotherapy is a localized therapy that can inhibit tumor growth, but may also affect and cause damage to surrounding tissues. Molecular targeting therapy has emerged as a new approach for treating cancer due to its characteristics such as high specificity and low toxicity. In terms of chemotherapy, numerous studies have demonstrated that inhibiting histone deacetylases shows good therapeutic effects for many cancers. Despite more than thirty years of studies, existing clinical candidate HDAC inhibitors still have problems such as poor pharmacokinetic properties, average efficacy and limited indications, and the combined advantages of HDAC targets have not been fully exploited. Moreover, the types of HDAC inhibitors on the market remain limited. Therefore, this target still has high research value and potential. Histone deacetylases (HDACs), discovered in the 1990s, are crucial for the post-translational modification of amino acid side chains in various proteins. They can enable the proteins to exist in many states and significantly change their biological properties. In light of the high expression of HDACs in a variety of tumor cells, HDAC inhibitors are used to effectively target tumor cells without causing significant toxicity. Meanwhile, the HDAC inhibitors can slow down and reduce the extent of the process by which tumor stem cells acquire drug resistance. Prostate cancer is the second most common cancer (second only to lung cancer) among men across the world. It commonly occurs in adult men over the age of 50. The prostate cancer is often curable before its metastasis. However, approximately 25% of prostate cancer patients suffer from metastasis. These patients often remain asymptomatic for months or even years in the early stages, making diagnosis and treatment more difficult. The prostate cancer could be initially treated by androgen deprivation, which, however, may easily lead to drug resistance, making it impossible to completely cure the prostate cancer. At present, studies have begun to explore the benefits of HDAC inhibitors for prostate cancer patients. First, the overexpression of HDAC6 is associated with the invasion and metastasis of tumor cells, and HDAC overexpression is often observed in prostate cancer patients. Second, HDAC shows high-level inhibition in a wide range of tumor systems. A considerable number of academic studies have provided experimental evidence for treating the prostate cancer using HDAC inhibitors. Meanwhile, the HDAC inhibitors can address the issue of resistance to many inhibitors. However, there is currently no HDAC inhibitor specifically for the prostate cancer.

### CONTENT OF THE INVENTION

In view of the defects existing in the prior art, an object of the present invention is to provide an imidazolidinedione-based HDAC inhibitor, a method for preparing the same, and use thereof.

The present invention solves the technical problem using the following technical solution: the imidazolidinedione-based HDAC inhibitor of the present invention is a substituted imidazolidinedione compound of Formula V below, or a stereisomer, hydrate or pharmaceutically acceptable salt thereof: 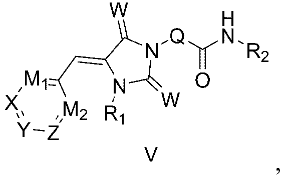 wherein in the formula, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₃, and R₃ may be hydrogen, alkyl, cyano, halogen, haloalkyl, hydroxy, mercapto, alkoxy, alkylthio, alkoxyalkyl, arylalkyl, aryl or Het;
Q is selected from the group consisting of a saturated or unsaturated straight or branched hydrocarbon group having a length of 1 to 9 carbon atoms, aryl and Het;
R₁ is hydrogen, deuterium, alkyl, haloalkyl, hydroxy, mercapto, alkoxy, alkylamino, alkylthio, alkoxyalkyl, methylene, arylalkyl, diarylalkyl, aryl or Het;
R₂ is selected from the group consisting of hydroxy, and 2-aminophenyl optionally substituted by one or more R₄;
R₄ is hydrogen, alkyl, cyano, halogen, haloalkyl, hydroxy, mercapto, alkoxy, alkylthio, alkoxyalkyl, arylalkyl, aryl or Het;
the alkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 6 carbon atoms;
the alkoxy is a straight or branched saturated hydrocarbon group having 1 to 9 carbon atoms, or a cyclic saturated hydrocarbon group having 3 to 9 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an oxygen atom; the alkylamino is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an NH atom;
the alkoxyalkyl represents linking the alkoxy defined as above to alkyl;
alkenyl or alkynyl is a straight or branched unsaturated hydrocarbon group having 1 to 9 carbon atoms and a double or triple bond;
the aryl is a carboatomic ring selected from the group consisting of phenyl, naphthyl, acenaphthenyl and tetrahydronaphthyl, each of which is optionally substituted by 1, 2 or 3 substituents, with each substituent independently selected from the group consisting of hydrogen, alkyl, cyano, halogen, haloalkyl, hydroxy, mercapto, alkoxy, alkylthio, alkoxyalkyl, arylalkyl, diarylalkyl, aryl and Het;
the arylalkyl or diarylalkyl represents linking the aryl defined as above to alkyl;
the Het is a monoheterocyclic ring selected from the group consisting of pyrrolyl, pyrazolyl, imidazolyl, furyl, thienyl, oxazolyl, isoxazolyl, thiazolyl, isothiazolyl, pyridinyl, pyrimidinyl, pyrazinyl and pyridazinyl, or is a biheterocyclic ring selected from the group consisting of quinolyl, quinoxalinyl, indolyl, benzoimidazolyl, benzooxazolyl, benzoisoxazolyl, benzothiazolyl, benzoisothiazolyl, benzofuryl, benzothienyl, 2,3-dihydrobenzo[1,4]dioxin and benzo[1,3]dioxol, or is selected from the group consisting of a monocyclic saturated hydrocarbon group having 3 to 6 carbon atoms, and a bicyclic saturated hydrocarbon group having 6 to 12 carbon atoms, wherein a carbon atom on a ring is independently and optionally substituted by 1 to 4 O, S, N or NH; and each monocyclic or bicyclic ring is optionally substituted by 1, 2 or 3 substituents, with each substituent independently selected from the group consisting of halogen, haloalkyl, hydroxy, alkyl and alkoxy.

The halogen is a substituent selected from the group consisting of fluorine, chlorine, bromine and iodine; and
the haloalkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated alkyl group having 1 to 6 carbon atoms, wherein one or more carbon atoms are substituted by one or more halogen atoms.

Preferably, a compound as below is further selected in the present invention as the imidazolidinedione-based HDAC inhibitor: in the Formula, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₃, and R₃ is hydrogen, alkyl, cyano, halogen, haloalkyl, alkoxy, alkylthio, or alkoxyalkyl;
Q is selected from the group consisting of a saturated or unsaturated straight or branched hydrocarbon group having a length of 1 to 9 carbon atoms, and aryl;
R₂ is selected from the group consisting of hydroxy, and 2-aminophenyl optionally substituted by one or more R₄; and
R₄ is hydrogen, alkyl, cyano, halogen, haloalkyl, aryl or Het.

Preferably, a compound as below is further selected in the present invention as the imidazolidinedione-based HDAC inhibitor: in the Formula, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₃, and R₃ is hydrogen, alkyl, halogen or alkoxy;
Q is selected from the group consisting of a saturated or unsaturated straight or branched hydrocarbon group having a length of 3 to 9 carbon atoms, and aryl;
R₂ is selected from the group consisting of hydroxy, and 2-aminophenyl optionally substituted by one or more R₄; and
R₄ is hydrogen, alkyl, cyano, halogen, haloalkyl, aryl or Het.

Preferably, in the inhibitor of Formula V, hydrogen attached to carbon is replaced by deuterium, which is an isotope of hydrogen.

Further preferably, the alkyl group is replaced by a deuterated alkyl group, the alkoxy group is replaced by a deuterated alkoxy group, the benzene ring is replaced by a deuterated benzene ring, and the aromatic ring is replaced by a deuterated aromatic ring.

preferably, the pharmaceutically acceptable salt refers to a salt form into which a basic group in the parent compound is converted, wherein the pharmaceutically acceptable salt is an inorganic or organic acid salt of a basic group, further preferably an amido or amino group; and the basic group in the parent compound reacts with 1 to 4 equivalents of an acid in a solvent system.

Preferably, the basic group of the compound of the present invention may form a salt with an acid, and the acid salt is specifically selected from: a salt formed with an inorganic acid, in particular a hydrohalic acid (such as hydrochloric acid, hydrobromic acid, or hydroiodic acid), nitric acid, sulfuric acid, phosphoric acid, carbonic acid, etc.; a salt formed with a lower alkylsulfonic acid, such as methanesulfonic acid or trifluoromethanesulfonic acid; a salt formed with an arylsulfonic acid, such as benzenesulfonic acid or p-toluenesulfonic acid; a salt formed with an organic acid, such as acetic acid, fumaric acid, tataric acid, oxalic acid, citric acid, maleic acid, malic acid or succinic acid; or a salt formed with an amino acid, such as aspartic acid or glutamic acid.

Preferably, the compound of the present invention and the pharmaceutically acceptable salt thereof further include a solvate or hydrate form.

Preferably, the structural formula of the compound in the imidazolidinedione-based HDAC inhibitor of the present invention includes isomeric forms, such as enantiomers, diastereomers, geometric isomers or conformational isomers, in particular R or S configurations containing asymmetric centers, (*Z*) or (*E*) isomers of double bonds, and (*Z*) or (*E*) conformational isomers.

Preferably, the inhibitor is one of:
(1) (Z)-N-hydroxy-7-(4-(2-methoxybenzylene)-2,5-dioxoimidazolidin-1-yl)heptanamide;
(2) (Z)-N-hydroxy-8-(4-(2-methoxybenzylene)-2,5-dioxoimidazolidin-1-yl)octanamide;
(3) (Z)-N-hydroxy-6-(4-(2-methoxybenzylene)-2,5-dioxoimidazolidazol-1-yl)hexanamide;
(4) (Z)-N-hydroxy-5-(4-(2 -methoxybenzylene)-2,5-dioxoimidazolidin-1-yl)pentanamide;
(5) (Z)-N-hydroxy-7-(4-(4-bromo-2-methoxybenzylene)-2,5-dioxoimidazolidin-1-yl)heptanamide;
(6) (Z)-N-hydroxy-8-(4-(4-bromo-2-methoxybenzylene)-2,5-dioxoimidazolidin-1-yl)octanamide;
(7) (Z)-N-hydroxy-9-(4-(2-methoxybenzylene)-2,5-dioxoimidazolidin-1-yl)nonanamide;
(8) (Z)-N-hydroxy-10-(4-(2-methoxybenzylene)-2,5-dioxoimidazolidin-1-yl)decanamide;
(9) (Z)-N-hydroxy-7-(4-(2-methoxybenzylene)-3-methyl-2,5-dioxoimidazolidin-1-yl)heptanamide;
(10) (Z)-N-hydroxy-7-(4-(4-bromo-2-methoxybenzylene)-3-methyl-2,5-dioxoimidazolidin-1-yl)heptanamide;
(11) (Z)-N-hydroxy-6-(4-(4-bromo-2-methoxybenzylene)-2,5-dioxoimidazolidin-1-yl)hexanamide;
(12) (Z)-N-hydroxy-6-(4-(3-methoxybenzylene)-2,5-dioxoimidazolidin-1-yl)hexanamide;
(13) (Z)-N-hydroxy-6-(4-(4-methoxybenzylene)-2,5-dioxoimidazolidin-1-yl)hexanamide;
(14) (Z)-N-hydroxy-6-(4-(2,4-dimethoxybenzylene)-2,5-dioxoimidazolidin-1-yl)hexanamide;
(15) (Z)-N-hydroxy-6-(4-(4-fluoro-2-methoxybenzylene)-2,5-dioxoimidazolidin-1-yl)hexanamide;
(16) (Z)-N-hydroxy-6-(4-(4-chloro-2-methoxybenzylene)-2,5-dioxoimidazolidin-1-yl)hexanamide;
(17) (Z)-N-hydroxy-6-(4-(2-methoxy-4-methylbenzylene)-2,5-dioxoimidazolidin-1-yl)hexanamide;
(18) (Z)-N-hydroxy-6-(4-(2-methoxy-4-(trifluoromethyl)benzylene)-2,5-dioxoimidazolidin-1-yl)hexanamide;
(19) (Z)-N-hydroxy-6-(2.5-dioxo-4-(3,4,5-trimethoxybenzylene)imidazolidin-1-yl)hexanamide;
(20) (Z)-N-hydroxy-6-(4-(naphth-1-ylmethylene)-2,5-dioxoimidazolidin-1-yl)hexanamide;
(21) (Z)-N-hydroxy-6-(4-(naphth-2-ylmethylene)-2,5-dioxoimidazolidin-1-yl)hexanamide;
(22) (Z)-N-hydroxy-6-(2,5-dioxo-4-(quinolin-2-ylmethylene)imidazolidin-1-yl)hexanamide;
(23) (Z)-N-hydroxy-6-(2,5-dioxo-4-(quinolin-3-ylmethylene)imidazolidin-1-yl)hexanamide;
(24) (Z)-N-hydroxy-6-(2,5-dioxo-4-(quinolin-4-ylmethylene)imidazolidin-1-yl)hexanamide;
(25) (Z)-N-hydroxy-6-(2,5-dioxo-4-(quinolin-6-ylmethylene)imidazolidin-1-yl)hexanamide;
(26) (Z)-N-hydroxy-6-(2,5-dioxo-4-(quinolin-5-ylmethylene)imidazolidin-1-yl)hexanamide;
(27) (Z)-N-hydroxy-6-(4-(furan-2-ylmethylene)-2,5-dioxoimidazolidin-1-yl)hexanamide;
(28) (Z)-N-hydroxy-6-(2,5-dioxo-4-(thiophen-2-ylmethylene)imidazolidin-1-yl)hexanamide;
(29) (Z)-N-hydroxy-6-(4-((2,3-dihydrobenzofuran-5-yl)methylene)-2,5-dioxoimidazolidin-1-yl)hexanamide;
(30) (Z)-N-hydroxy-6-(4-(benzofuran-5-ylmethylene)-2,5-dioxoimidazolidin-1-yl)hexanamide;
(31) (Z)-N-hydroxy-6-(4-(benzo[d][l,3]dioxol-5-ylmethylene)-2,5-dioxoimidazolidin-1-yl)hexanamide;
(32) (Z)-N-hydroxy-6-(4-((2,2-difluorobenzo[d][1,3]dioxol-5-yl)methylene)-2,5-dioxoimidazolidin-1-yl)hexanamide;
(33) (Z)-N-(2-amino-4-fluorophenyl)-4-((2,5-dioxo-4-(pyridin-3-ylmethylene)imidazolidin-1-yl)methyl)benzamide;
(34) (Z)-N-(2-amino-4-fluorophenyl)-4-((2,5-dioxo-4-(pyridin-3-ylmethylene)imidazolidin-1-yl)methyl)benzamide; and
(35) (Z)-N-hydroxy-6-(2,5-dioxo-4-(4-(pyridin-4-yl)quinolin-6-yl)methylene)imidazolidin-1-yl)hexanamide.

A first method for preparing the inhibitor described above includes: subjecting substituted formaldehyde (A) and hydantoin or substituted hydantoin (B), as starting materials, to a Knoevenagel condensation reaction under the action of alanine to obtain an intermediate (C); subjecting the intermediate (C) and bromocarboxylate to a nucleophilic substitution reaction to obtain a key intermediate (D); hydrolyzing the intermediate (D) under acidic conditions to obtain an intermediate (E); and condensing the intermediate (E) with hydroxylamine hydrochloride to obtain a target compound (1-32), with a synthesis route as below:

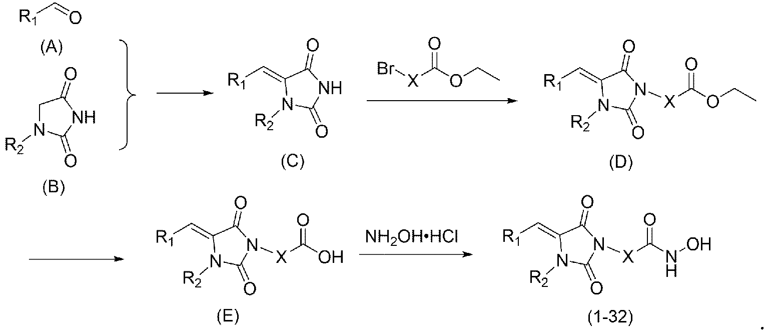

A second method for preparing the inhibitor described above includes: subjecting substituted formaldehyde (A) and hydantoin or substituted hydantoin (B), as starting materials, to a Knoevenagel condensation reaction under the action of alanine to obtain an intermediate (C); subjecting the intermediate (C) and bromocarboxylate to a nucleophilic substitution reaction to obtain a key intermediate (D); hydrolyzing the intermediate (D) under acidic conditions to obtain an intermediate (E); and condensing the intermediate (E) with 4-fluoro-1,2-phenylenediamine to obtain a target compound (1-32), with a synthesis route as below:

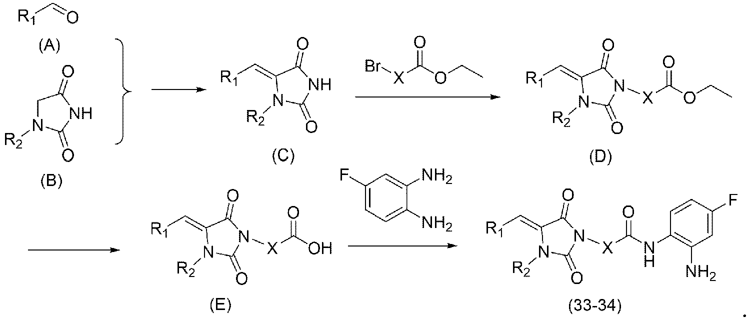

A further method for preparing the inhibitor described above includes: subjecting a compound (F), as a starting material, and cycl-isopropylidene malonate to a Combes quinoline synthesis reaction to obtain an intermediate (G); subjecting the intermediate (G) and phosphorus tribromide to a bromination reaction in DMF to obtain an intermediate (H); subjecting the intermediate (H) and 4-pyridineboronic acid to a Suzuki coupling reaction to obtain an intermediate (I); and subjecting the intermediate (I) and lithium aluminum hydride to a reduction reaction to obtain an intermediate (J); subjecting the intermediate (J) to selective oxidation to obtain a key intermediate (K); and subjecting the intermediate (K) to four sequential reactions to obtain a target compound (35), with a synthesis route as below:

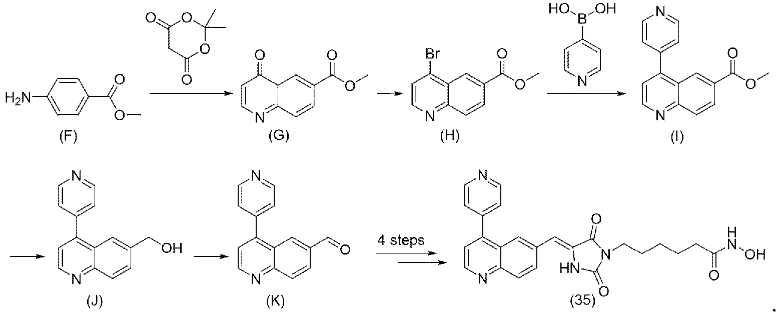

Use of the imidazolidinedione-based HDAC inhibitor described above in the preparation of a medicament for inhibiting growth of cancer cells is provided.

Due to low HDAC inhibitory activity and insufficient anti-tumor effect, HDAC is selected as the lead compound for further structural modification. It is determined that imidazolidinedione is used as a basic skeleton structure and substituent groups are screened, to bring about an efficient inhibitory effect.

Compared with the prior art, the present invention has the following significant advantages: (1) efficient and excellent HDAC inhibitory activity; (2) low cost, good efficacy, low toxicity and high yields of intermediates during synthesis, resulting in reduced resource waste and further reduced cost; and (3) significant anti-tumor activity and low dosage requirement.

### SPECIFIC IMPLEMENTATIONS

The technical solutions of the present invention are further described below in conjunction with examples.

Reagents were commercially available from commercial suppliers such as Anhui Zesheng Technology Co., Ltd., J&K Scientific, Aladdin Reagent Co., Ltd., Beijing Ouhe Technology Co., Ltd., etc., and were used without further purification, unless otherwise indicated. General reagents were commercially available from Xilong Chemical Co., Ltd., Nanjing Chemical Reagent Co., Ltd., Sinopharm Chemical Reagent Co., Ltd., Qingdao Haiyang Chemical Co., Ltd., etc. In the examples, all temperatures were set in degrees Celsius, unless otherwise indicated. In the examples described below, chromatographic purification was performed using silica gel columns (200-300 mesh) commercially available from Qingdao Haiyang Chemical Co., Ltd. Nuclear magnetic resonance (NMR) spectra were recorded in a CDCl₃ or DMSO-*d₆* solvent (in ppm), using tetramethylsilane (TMS) at 0 ppm as internal reference. Peak multiplicities were denoted in abbreviation as follows: s (singlet), d (doublet), t (triplet), m (multiplet), br (broadened), dd (doublet of doublets), dt (doublet of triplets). Coupling constants were expressed in hertz (Hz).

In the examples described below, low-resolution mass spectrometry (MS) data were analyzed using a G1329B autosampler and a G4212B detector in an Agilent 6120 series LC-MS equipped with a G1311B quaternary pump and a G1316A column oven.

In the examples described below, for ease of description, some starting materials will be described by their abbreviations, which are illustrated below in contrast with their full names: DCM denotes CH₂Cl₂, i.e., dichloromethane; CDCl₃ denotes deuterated chloroform; PE denotes petroleum ether; both EtOAc and EA denote ethyl acetate; both MeOH and CH₃OH denote methanol; ClSO₃H denotes chlorosulfonic acid; both TEA and Et₃ denote triethylamine; DMSO-*d₆* denotes hexadeuterated dimethyl sulfoxide; THF denotes tetrahydrofuran; NaCl denotes sodium chloride; Na₂SO₄ denotes sodium sulfate; and DIPEA denotes N,N-diisopropylethylamine, PyBOP denotes 1H-benzotriazol-1-yloxotripyrrolidinyl hexafluorophosphate, and Pd(dppf)Cl₂ denotes a 1,1'-Bis(diphenylphosphino)ferrocene-palladium(II)dichloride dichloromethane complex.

### Example 1

Synthesis of (Z)-N-hydroxy-7-(4-(2-methoxybenzylene)-2,5-dioxoimidazolidin-1-yl)heptanamide, with synthesis steps as below.

Step 1: Synthesis of (Z)-5-(2-methoxybenzylene)imidazolidinyl-2,4-dione, with a structural formula as below: 1.11 g of 2-methoxybenzaldehyde (10 mmol), 1.36 g of hydantoin (11.0 mmol, 1.1 eq.) and 1.07 g of β-alanine (12.0 mmol, 1.2 eq.) were weighed and dissolved in 25 mL of acetic acid, and then added to 100 mL of an eggplant-shaped reaction flask for a reaction to proceed overnight under reflux at 120 °C, which was monitored by thin-layer chromatography (TLC) spotting application until full consumption of starting materials. A reaction mixture was slowly added to ice water and stirred at room temperature for precipitation of a large amount of solids, followed by filtering to obtain a crude product, and the crude product was purified by silica gel column chromatography (PE:EA = 1:1). Light yellow solid, yield: 76%. ¹H NMR (300 MHz, Chloroform-*d*) *δ* 9.62 (s, 1H), 9.05 (s, 1H), 7.67 (dd, *J* = 7.8, 1.6 Hz, 1H), 7.37 (td, *J* = 7.8, 1.5 Hz, 1H), 7.07 (td, *J* = 7.6, 1.2 Hz, 1H), 7.00 (dd, *J* = 8.1, 1.3 Hz, 1H), 6.88 (s, 1H), 3.87 (s, 2H). Step 2: Synthesis of ethyl (Z)-7-(4-(2-methoxybenzylene)-2,5-dioxoimidazolidin-1-yl)heptanoate, with a structural formula as below: 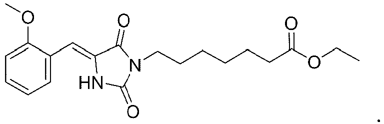 5 mmol (Z)-5-(2-methoxybenzylene)imidazolidinyl-2.4-dione and 10 mmol potassium carbonate (2.0 eq.) were weighed and dissolved in 20 mL of DMF, cooled to 0 °C and stirred for 30 min, followed by slow dropwise addition of 6 mmol ethyl 7-bromoheptanoate (1.2 eq.), and a resulting mixture was heated to room temperature to allow a reaction to proceed for 6-8 h. Upon completion of the reaction, a residue was concentrated and purified by column chromatography (PE:EA = 5:1 to 2:1). White solid, yield: 52%. ¹H NMR (400 MHz, Chloroform-*d₆*) *δ* 8.00 (s, 1H), 7.41-7.33 (m, 2H), 7.08-6.95 (m, 2H), 6.76 (s, 1H), 4.13q, *J* = 7.1 Hz, 2H), 3.96 (s, 3H), 3.61 (dd, *J* = 8.0, 6.6 Hz, 2H), 2.30 (t, *J* = 7.5 Hz, 2H), 1.76 (s, 1H), 1.75-1.58 (m, 4H), 1.43-1.33 (m, *J* = 4.0, 3.5 Hz, 4H), 1.26 (t, *J* = 7.1 Hz, 4H).

Step 3: Synthesis of (Z)-N-hydroxy-7-(4-(2-methoxybenzylene)-2,5-dioxoimidazolidin-1-yl)heptanamide, with a structural formula as below: 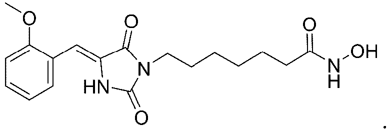 First, (Z)-7-(4-(2-methoxybenzylene)-2,5-dioxoimidazolidin-1-yl)heptanoic acid was prepared as follows: 5 mmol ethyl ((Z)-7-(4-(2-methoxybenzylene)-2,5-dioxoimidazolidin-1-yl)heptanoate was dissolved in 10 mL of an acetic acid solution and then added to a 50 mL eggplant-shaped flask; 1 mL of concentrated hydrochloric acid was volumetrically weighed and slowly added dropwise to the flask; the reactants were heated to 90 °C to allow a reaction to proceed for 6 h; upon completion of the reaction, the reaction mixture was added to ice saline while it was still hot, for precipitation of a large amount of white solids; and the white solids were dried to obtain crude (Z)-7-(4-(2-methoxybenzylene)-2,5-dioxoimidazolidin-1-yl)heptanoic acid. 1 mmol crude (Z)-7-(4-(2-methoxybenzylene)-2,5-dioxoimidazolidin-1-yl)heptanoic acid and 1.5 mmol PyBOP condensing agent (1.5 eq.) were added to a 25 mL reaction flask; 6 mL of a DMF solution was added; 590 µL of DIPEA (3.5 eq.) was added to the mixture; and the mixture was stirred at room temperature for several minutes, followed by the addition of 0.14 g of hydroxylamine hydrochloride (2.0 eq.), allowing a reaction to proceed for 5 h at room temperature, which was monitored by TLC until full consumption of the intermediate (DCM:MeOH = 25:1); and the reaction mixture was concentrated in vacuum and then purified by column chromatography. White solid, yield: 53%. ¹H NMR (400 MHz, DMSO-*d₆*) *δ* 10.60 (d, *J* = 3.5 Hz, 1H), 10.32 (d, *J* = 3.5 Hz, 1H), 8.52 (s, 1H), 7.66 (dd, *J* = 7.7, 1.6 Hz, 1H), 7.37 (td, *J* = 7.8, 1.6 Hz, 1H), 7.07 (td, *J* = 7.6, 1.2 Hz, 1H), 7.00 (dd, *J* = 8.0, 1.3 Hz, 1H), 6.86 (s, 1H), 3.87 (s, 2H), 3.72 (t, *J* = 6.6 Hz, 2H), 1.99 (t, *J* = 8.6 Hz, 2H), 1.62 (p, *J* = 6.7 Hz, 2H), 1.55 (ddd, *J* = 16.3, 8.5, 7.4 Hz, 2H), 1.41-1.28 (m, 4H); ¹³C NMR (101 MHz, DMSO-*d₆*) *δ* 174.79, 163.98, 158.56, 154.37, 132.25, 129.69, 127.13, 124.90, 123.38, 112.63, 110.42, 55.63, 37.80, 32.04, 28.24, 27.26, 26.86, 25.05.

### Example 2

(Z)-N-hydroxy-8-(4-(2-methoxybenzylene)-2.5-dioxoimidazolidin-1-yl)octanamide, with a structural formula as below: 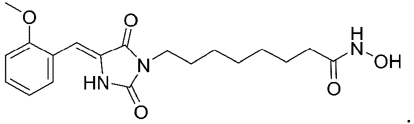 The fragment ethyl 7-bromoheptanoate in Step 2 of Example 1 was replaced with ethyl 8-bromooctanoate, while other steps and procedures were identical to those in Example 1. White solid, yield: 43%. ¹H NMR (400 MHz, DMSO-*d₆*) *δ* 10.45 (d, *J* = 3.5 Hz, 1H), 9.08 (d, *J* = 3.5 Hz, 1H), 8.52 (s, 1H), 7.62 (dd, *J* = 7.7, 1.6 Hz, 1H), 7.34 (td, *J* = 7.9, 7.3, 1.6 Hz, 1H), 7.09-6.95 (m, 2H), 6.74 (s, 1H), 3.85 (s, 3H), 3.46 (t, *J* = 7.1 Hz, 2H), 1.93 (t, *J* = 7.4 Hz, 2H), 1.51 (dp, *J* = 29.4, 7.2 Hz, 4H), 1.25 (h, *J* = 6.0 Hz, 6H); ¹³C NMR (101 MHz, DMSO-*d₆*) *δ* 174.80, 163.98, 158.56, 154.37, 132.25, 129.69, 127.13, 124.90, 123, 38, 112.63, 110.42, 55.63, 37.80, 32.04, 28.81, 27.94, 27.26, 27.07, 25.05.

### Example 3

(Z)-N-hydroxy-6-(4-(2-methoxybenzylene)-2,5-dioxoimidazolidazol-1-yl)hexanamide, with a structural formula as below: 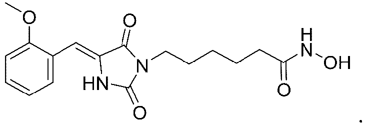 The fragment ethyl 7-bromoheptanoate in Step 2 of Example 1 was replaced with ethyl 6-bromohexanoate, while other steps and procedures were identical to those in Example 1. White solid, yield: 47%. ¹H NMR (400 MHz, DMSO-*d₆*) *δ* 10.45 (d, *J* = 3.5 Hz, 1H), 8.69 (d, *J* = 3.7 Hz, 1H), 7.61 (dd, *J* = 7.7.1.6 Hz, 1H), 7.34 (ddd, *J* = 8.7, 7.4, 1.6 Hz, 1H), 7.05 (dd, *J* = 8.4, 1.1 Hz, 1H), 6.98 (td, *J* = 7.4, 1.0 Hz, 1H), 6.73 (s, 1H), 3.84 (s, 3H), 3.44 (t, *J* = 7.11 Hz, 3H), 2.01-1.89 (m, 2H), 1.51 (dq, *J* = 15.1, 7.5 Hz, 4H), 1.30-1.13 (m, 2H); ¹³C NMR (101 MHz, DMSO-*d₆*) *δ* 169.72, 164.59, 157.59, 155.51, 130.87, 129.71, 126.96, 121.62, 121.08, 111.59, 104.57, 56.07, 38.28, 32.50, 27.81, 26.15, 24.61.

### Example 4

(Z)-N-hydroxy-5-(4-(2-methoxybenzylene)-2,5-dioxoimidazolidin-1-yl)pentanamide, with a structural formula as below: 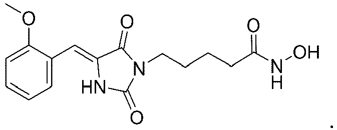 The fragment ethyl 7-bromoheptanoate in Step 2 of Example 1 was replaced with ethyl 5-bromopentanoate, while other steps and procedures were identical to those in Example 1. White solid, yield: 48%. ¹H NMR (400 MHz, DMSO-*d₆*) *δ* 10.36 (d, *J* = 3.5 Hz, 1H), 9.08 (d, *J* = 3.5 Hz, 1H), 8.52 (s, 1H), 7.61 (dd, *J* = 7.7, 1.6 Hz, 1H), 7.35 (ddd, *J* = 8.7, 7.4, 1.6 Hz, 1H), 7.06 (dd, *J* = 8.4, 1.0 Hz, 1H), 6.99 (td, *J* = 7.5, 1.0 Hz, 1H), 6.74 (s, 1H), 3.85 (s, 3H), 3.46 (t, *J* = 6.5 Hz, 2H), 1.98 (t, *J* = 6.2 Hz, 2H), 1.50 (ddt, *J* = 15.7, 12.1, 8.2 Hz, 4H); ¹³C NMR (101 MHz, DMSO-*d₆*)*δ* 170.22, 164.78, 157.53, 155.60, 131.19, 129.64, 126.68, 121.34, 121.20, 111.63, 105.46, 56.02, 38.13, 32.13, 27.54, 22.82.

### Example 5

(Z)-N-hydroxy-7-(4-(4-bromo-2-methoxybenzylene)-2,5-dioxoimidazolidin-1-yl)heptanamide, with a structural formula as below:
The starting material 2-methoxybenzaldehyde in Step 1 of Example 1 was replaced with ethyl 4-bromo-2-methoxybenzaldehyde, while other steps and procedures were identical to those in Example 1. White solid, yield: 38%. ¹H NMR (400 MHz, DMSO-*d₆*) *δ* 10.60 (d, *J* = 3.5 Hz, 1H), 10.32 (d, *J* = 3.5 Hz, 1H), 8.51 (s, 1H), 7.52 (d, *J* = 9.0 Hz, 1H), 7.40 (dd, *J* = 8.8, 2.2 Hz, 1H), 7.23 (d, *J* = 2.2 Hz, 1H), 6.85 (s, 1H), 3.88 (s, 2H), 3.72 (t, *J* = 6.6 Hz, 2H), 1.99 (t, *J* = 8.6 Hz, 2H), 1.62 (p, *J* = 6.7 Hz, 2H), 1.55 (ddd, *J* = 16.3, 8.5, 7.4 Hz, 2H), 1.41-1.28 (m, 4H); ¹³C NMR (101 MHz, DMSO-*d₆*) *δ* 178.26, 162.10, 156.21, 152.45, 135.20, 124.25, 123.56, 123.21, 120.64, 112.11, 111.87, 56.07, 36.50, 32.64, 27.97, 27, 02, 26.66, 25.14.

### Example 6

(Z)-N-hydroxy-8-(4-(4-bromo-2-methoxybenzylene)-2.5-dioxoimidazolidin-1-yl)octanamide, with a structural formula as below: 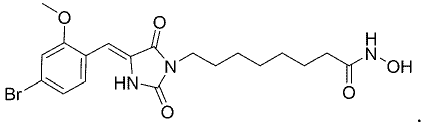 The starting material 2-methoxybenzaldehyde in Step 1 of Example 1 was replaced with 4-bromo-2-methoxybenzaldehyde, and the fragment ethyl 7-bromoheptanoate in Step 2 was replaced with ethyl 8-bromooctanoate, while other steps and procedures were identical to those in Example 1. White solid, yield: 35%. ¹H NMR (400MHz, DMSO-*d₆*) *δ* 10.58 (d, *J* = 3.5 Hz, 1H), 10.30 (d, *J* = 3.5 Hz, 1H), 8.53 (s, 1H), 7.50 (d, *J* = 9.0 Hz, 1H), 7.42 (dd, *J* = 8.8, 2.2 Hz, 1H), 7.21 (d, *J* = 2.2 Hz, 1H), 6.83 (s, 1H), 3.76 (s, 2H), 3.70 (t, *J* = 6.6 Hz, 2H), 2.01 (t, *J* = 8.6 Hz*,* 2H), 1.66- 1.49 (m, 4H), 1.43-1.23 (m, 7H); ¹³C NMR (101 MHz, DMSO-*d₆*) *δ* 169.66, 164.44, 158.25, 155.49, 131.06, 127.61, 123, 88, 123.25, 121.16, 114.92, 103, 14, 56.67, 38.40, 32.68, 28.91, 28.69, 28.01, 26.52, 25.51.

### Example 7

(Z)-N-hydroxy-9-(4-(2-methoxybenzylene)-2.5-dioxoimidazolidin-1-yl)nonanamide, with a structural formula as below: 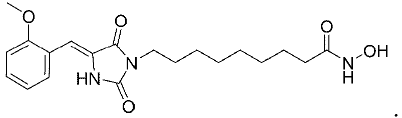 The fragment ethyl 7-bromoheptanoate in Step 2 of Example 1 was replaced with ethyl 9-bromononanoate, while other steps and procedures were identical to those in Example 1. White solid, yield: 39%. ¹H NMR (400 MHz, DMSO-*d₆*) *δ* 10.62 (d, *J* = 3.5 Hz, 1H), 10.32 (d, *J* = 3.5 Hz, 1H), 8.66 (s, 1H), 7.60 (dd, *J* = 7.6, 1.6 Hz, 1H), 7.39-7.29 (m, 1H), 7.05 (d, *J* = 8.3 Hz, 1H), 6.98 (t, *J* = 7.5 Hz, 1H), 6.73 (s, 1H), 3.84 (s, 3H), 3.45 (t, *J* = 7.1 Hz, 2H), 1.92 (t, *J* = 7.3 Hz, 2H), 1.55 (q, *J* = 7.0 Hz, 2H), 1.46 (p, *J* = 7.5, 7.0 Hz, 2H), 1.28-1.20 (m, 8H); ¹³C NMR (101 MHz, DMSO-*d₆*) *δ* 169.71, 164.55, 157.61, 155.50, 130.79, 129.74, 126.98, 121.68, 121.05, 111.59, 104.42, 56.08, 38.35, 32.68, 29.05, 28.96, 28.89, 28.01, 27.91, 26.58, 25.55.

### Example 8

(Z)-N-hydroxy-10-(4-(2-methoxybenzylene)-2.5-dioxoimidazolidin-1-yl)decanamide, with a structural formula as below: 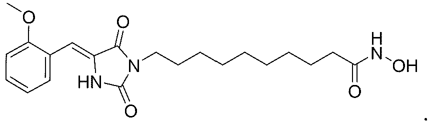 The fragment ethyl 7-bromoheptanoate in Step 2 of Example 1 was replaced with ethyl 10-bromodecanoate, while other steps and procedures were identical to those in Example 1. White solid, yield: 32%. ¹H NMR (400 MHz, DMSO-*d₆*) *δ* 10.63 (d, *J* = 3.5 Hz, 1H), 10.32 (d, *J* = 3.5 Hz, 1H), 8.66 (s, 1H), 7.60 (dd, *J* = 7.7, 1.6 Hz, 1H), 7.39-7.29 (m, H), 7.05 (d, *J* = 8.3 Hz, 1H), 6.98 (t, *J* = 7.5 Hz, 1H), 6.73 (s, 1H), 3.84 (s, 3H), 3.45 (t, *J* = 7. Hz, 2H), 1.92 (t, *J* = 7.4 Hz, 2H), 1.50 (dp, *J* = 33.1, 7.0 Hz, 4H), 1.24 (d, *J* = 8.0 Hz, 10H); ¹³C NMR (101 MHz, DMSO-*d₆*) *δ* 169.64, 164.54, 157.62, 155.49, 130.76, 129.75, 127.00, 121.71, 121.04, 111.58, 104.35, 56.08, 38.35, 32.71, 29.27, 29.14, 29.01, 28.96, 28.01, 26.71, 25.57.

### Example 9

(Z)-N-hydroxy-7-(4-(2-methoxybenzylene)-3-methyl-2,5-dioxoimidazolidin-1-yl)heptanamide, with a structural formula as below: 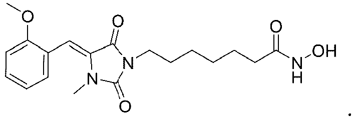 The starting material hydantoin in Step 1 of Example 1 was replaced with 1-methyl hydantoin, while other steps and procedures were identical to those in Example 1. White solid, yield: 34%. ¹H NMR (400 MHz, DMSO-*d₆*) *δ* 10.62 (d, *J* = 3.5 Hz, 1H), 10.32 (d, *J* = 3.5 Hz, 1H), 52s, 1H), 7.71 (dd, *J* = 7.7, 1.6 Hz, 1H), 7.37 (td, *J* = 7.8, 1.6 Hz, 1H), 7.36 (s, 1H), 7.07 (td, *J* = 7.6, 1.2 Hz, 1H), 7.00 (dd, *J* = 8.1, 1.3 Hz, 1H), 3.87 (s, 2H), 3.72 (t, *J* = 6.7 Hz, 2H), 3.30 (s, 2H), 1.99 (t, *J* = 8.6 Hz, 2H), 1.66-1.50 (m, 4H), 1.41-1.28 (m, 4H); ¹³C NMR (101 MHz, DMSO-*d₆*) *δ* 169.54, 163.56, 161.67, 157.47, 131.35, 131.26, 130.75, 129.45, 121.50, 120.34, 111.34, 56.02, 38.90, 32.63, 30.09, 28.57, 27.96, 26.65, 25.43.

### Example 10

((Z) -N-hydroxy-7- (4- (4-bromo-2-methoxybenzylene) -3-methyl-2, 5-dioxoimidazolidin-1-yl) heptanamide, with a structural formula as below: 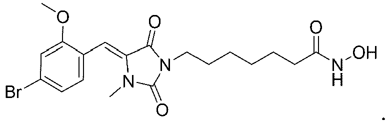 The starting material hydantoin in Step 1 of Example 1 was replaced with 1-methylhydantoin, and 2-methoxybenzaldehyde was replaced with 4-bromo-2-methoxybenzaldehyde, while other steps and procedures were identical to those in Example 1. White solid, yield: 35%, ¹H NMR (400 MHz, DMSO-*d₆*) *δ* 10.34 (d, *J* = 3.3 Hz, 1H), 8.67 (d, *J* = 3.5 Hz, 1H), 7.88 (d, *J* = 9.0 Hz, 1H), 7.24 (s, 1H), 7.13 (dd, *J* = 9.0, 2.2 Hz, 1H), 7.11 (d, *J* = 2.2 Hz, 1H), 3.88 (s, 2H), 33.72 (t, *J* = 6.7 Hz, 2H), 3.30 (s, 2H), 1.99 (t, *J* = 8.6 Hz, 2H), 1.66-1.50 (m, 4H), 1.41-1.28 (m, 4H); ¹³C NMR (101 MHz, DMSO-*d₆*)*δ* 169.57, 163.47, 158.56, 153.56, 132.71, 130.04, 123.28, 123.05, 120.99, 114.72, 114.43, 56.60, 38.93 ,32.63, 30.14, 28.56, 27.93, 26.67, 25.41.

### Example 11

(Z)-N-hydroxy-6-(4-(4-bromo-2-methoxybenzylene)-2,5-dioxoimidazolidin-1-yl)hexanamide, with a structural formula as below: 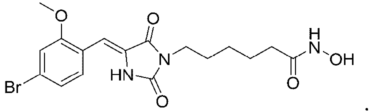 The starting material 2-methoxybenzaldehyde in Step 1 of Example 1 was replaced with 4-bromo-2-methoxybenzaldehyde, and the fragment ethyl 7-bromoheptanoate in Step 2 was replaced with ethyl 6-bromohexanoate, while other steps and procedures were identical to those in Example 1. White solid, yield: 40%. ¹H NMR (400 MHz, DMSO-*d₆*) *δ* 10.60 (d, *J* = 3.5 Hz, 1H), 8.76 (d, *J* = 3.5 Hz, 1H), 8.43 (s, 1H), 7.51 (d, *J* = 9.0 Hz, 1H), 7.30 (dd, *J* = 9.0, 2.0 Hz, 1H), 7.15 (d, *J* = 1.9 Hz, 1H), 6.75 (s, 1H), 3.98 (s, 2H), 3.74 (t, *J* = 6.6 Hz, 2H), 2.10 (t, *J* = 8.6 Hz*,* 2H), 1.69-1.54 (m, 4H), 1.45-1.29 (m, 2H); ¹³C NMR (101 MHz, DMSO-*d₆*) *δ* 169.80, 165.71, 158.87, 155.31, 134.44, 127.54, 124.90, 124.46, 122.70, 118.04, 112.23, 57.17, 36.98, 33.77, 27.55, 26.54, 24.60.

### Example 12

(Z)-N-hydroxy-6-(4-(3-methoxybenzylene)-2,5-dioxoimidazolidazol-1-yl)hexanamide, with a structural formula as below: 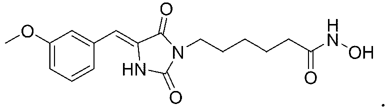 The starting material 2-methoxybenzaldehyde in Step 1 of Example 1 was replaced with 3-methoxybenzaldehyde, and the fragment ethyl 7-bromoheptanoate in Step 2 was replaced with ethyl 6-bromohexanoate, while other steps and procedures were identical to those in Example 1. White solid, yield: 39%. ¹H NMR (400 MHz, DMSO-*d₆*) *δ* 10.62 (d, *J* = 3.3 Hz, 1H), 8.92 (d, *J* = 3.5 Hz, 1H), 8.79 (s, 1H), 7.26 (ddd, *J* = 8.0, 7.0, 1.0 Hz, 1H), 7.15 (dt, *J* = 8.1.2.5 Hz, 1H), 7.02- 6.96 (m, 2H), 6.65 (s, 1H), 3.82 (s, 2H), 3.72 (t, *J* = 6.6 Hz, 2H), 1.99 (t, *J* = 8.6 Hz, 2H), 1.68-1.56 (m, 4H), 1.44-1.29 (m, 2H); ¹³C NMR (101 MHz, DMSO-*d₆*) *δ* 169.20, 160.57, 159.87, 155.68, 134.71, 130.75, 126.30, 124.62, 119.25, 118.51, 116.35, 52.27, 37.70, 36.52, 27.81, 26.68, 25.41.

### Example 13

(Z)-N-hydroxy-6-(4-(4-methoxybenzylene)-2,5-dioxoimidazolidazol-1-yl)hexanamide, with a structural formula as below: 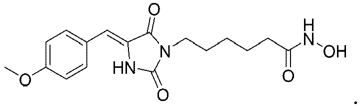 The starting material 2-methoxybenzaldehyde in Step 1 of Example 1 was replaced with 4-methoxybenzaldehyde, and the fragment ethyl 7-bromoheptanoate in Step 2 was replaced with ethyl 6-bromohexanoate, while other steps and procedures were identical to those in Example 1. White solid, yield: 38%. ¹H NMR (400 MHz, DMSO-*d₆*) *δ* 10.56 (d, *J* = 3.3 Hz, 1H) , 9.20 (d, *J* = 3.5 Hz, 1H), 8.82 (s, 1H), 7.56-7.50 (m, 2H), 7.08-7.01 (m, 2H), 6.59 (s, 1H), 3.82 (s, 2H), 3.72 (t, *J* = 6.6 Hz, 2H), 1.99 (t, *J* = 8.6 Hz, 2H), 1.67-1.52 (m, 4H), 1.42-1.33 (m, 2H); ¹³C NMR (101 MHz, DMSO-*d₆*) *δ* 170.80, 168.10, 164.70, 150.38, 134.53, 129.69, 125.16, 119.05, 114.85, 56.32, 36.80, 31.22, 27.21, 26.48, 24.81.

### Example 14

(Z)-N-hydroxy-6-(4-(2,4-dimethoxybenzylene)-2.5-dioxoimidazolidin-1-yl)hexanamide, with a structural formula as below: 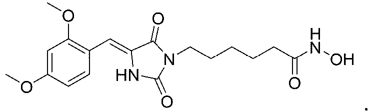 The starting material 2-methoxybenzaldehyde in Step 1 of Example 1 was replaced with 2.4-dimethoxybenzaldehyde, and the fragment ethyl 7-bromoheptanoate in Step 2 was replaced with ethyl 6-bromohexanoate, while other steps and procedures were identical to those in Example 1. White solid, yield: 42%. ¹H NMR (400 MHz, DMSO-*d₆*) *δ* 10.52 (d, *J* = 3.5 Hz, 1H), 9.02 (d, *J* = 3.5 Hz, 1H), 8.56 (s, 1H), 7.53 (d, *J* = 8.2 Hz, 1H), 6.74 (s, 1H), 6.63-6.56 (m, 2H), 3.88 (s, 2H), 3.82 (s, 2H), 3.72 (t, *J* = 6.6 Hz, 2H), 1.99 (t, *J* = 8.6 Hz, 2H), 1.64-1.50 (m, 4H), 11.47-1.32 (m, 2H); ¹³C NMR (101 MHz, DMSO-*d₆*) *δ* 169.57, 164.50, 160.87, 154.92, 152.61, 135.81, 129.45, 121.12, 115.45, 110.55, 100.21, 58.67, 53.12, 39.71, 33.55, 29.45, 26.53, 25.12.

### Example 15

(Z)-N-hydroxy-6-(4-(4-fluoro-2-methoxybenzylene)-2.5-dioxoimidazolidin-1-yl)hexanamide, with a structural formula as below: 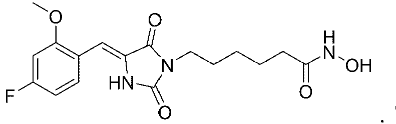 The starting material 2-methoxybenzaldehyde in Step 1 of Example 1 was replaced with 4-fluoro-2-methoxybenzaldehyde, and the fragment ethyl 7-bromoheptanoate in Step 2 was replaced with ethyl 6-bromohexanoate, while other steps and procedures were identical to those in Example 1. Light yellow solid, yield: 40%. ¹H NMR (400 MHz, DMSO-*d₆*) *δ* 10.54 (d, *J* = 3.5 Hz, 1H), 9.08 (d, *J* = 3.5 Hz, 1H), 8.51 (s, 1H), 7.52 (dd, *J* = 8.7, 5.0 Hz, 1H), 7.00 (ddd, *J* = 8.8, 8.1, 1.9 Hz, 1H), 6.81-6.76 (m, 2H), 3.77 (s, 2H), 3.54 (t, *J* = 6.6 Hz, 2H), 2.00 (t, *J* = 8.6 Hz, 2H), 1.64-1.50 (m, 4H), 1.39-1.29 (m, 2H); ¹³C NMR (101 MHz, DMSO-*d₆*) *δ* 169.40, 165.87, 164.98, 163.92, 160.22, 157.95, 153.67, 131.80, 131.21, 128.05, 122.61, 120.67, 113.06, 111.05, 109.97, 101.65, 101.42, 52.05, 38.51, 33.34, 28.05, 27.48, 25.21.

### Example 16

(Z)-N-hydroxy-6-(4-(4-chloro-2-methoxybenzylene)-2,5-dioxoimidazolidin-1-yl)hexanamide, with a structural formula as below: 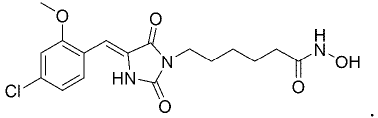 The starting material 2-methoxybenzaldehyde in Step 1 of Example 1 was replaced with 4-chloro-2-methoxybenzaldehyde, and the fragment ethyl 7-bromoheptanoate in Step 2 was replaced with ethyl 6-bromohexanoate, while other steps and procedures were identical to those in Example 1. White solid, yield: 36%. ¹H NMR (400 MHz, DMSO-*d₆*) *δ* 10.62 (d, *J* = 3.5 Hz, 1H), 9.08 (d, *J* = 3.5 Hz, 1H), 8.60 (s, 1H), 7.54 (d, *J* = 8.8 Hz, 1H), 7.34-7.28 (m, 1H), 7.02 (d, *J* = 1.9 Hz, 1H), 6.82 (s, 1H), 3.98 (s, 2H), 3.70 (t, *J* = 6.6 Hz, 2H), 1.98 (t, *J* = 8.6 Hz, 2H), 1.64-1.50 (m, 4H), 1.41-1.30 (m, 2H); ¹³C NMR (101 MHz, DMSO-*d₆*) *δ* 169.50, 166.58, 160.54, 159.70, 136.08, 132.87, 128.71, 124.33, 120.71, 111.45, 110.12, 52.10, 38.70, 35.44, 29.02, 27.52, 25.21.

### Example 17

(Z)-N-hydroxy-6-(4-(2-methoxy-4-methylbenzylene)-2.5-dioxoimidazolidin-1-yl)hexanamide, with a structural formula as below: 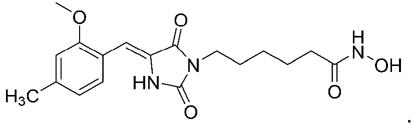 The starting material 2-methoxybenzaldehyde in Step 1 of Example 1 was replaced with 2-methoxy-4-methylbenzaldehyde, and the fragment ethyl 7-bromoheptanoate in Step 2 was replaced with ethyl 6-bromohexanoate, while other steps and procedures were identical to those in Example 1. White solid, yield: 41%. ¹H NMR (400 MHz, DMSO-*d₆*) *δ* 10.61 (d, *J* = 3.5 Hz, 1H), 8.86 (d, *J* = 3.5 Hz, 1H), 8.53 (s, 1H), 7.66 (dd, *J* = 7.7, 1.6 Hz, 1H), 7.31 (td, *J* = 7.8, 1.6 Hz, 1H), 7.09 (td, *J* = 7.6, 1.2 Hz, 1H), 7.00 (dd, *J* = 8.0, 1.3 Hz, 1H), 6.84 (s, 1H), 3.89 (s, 2H), 3.70 (t, *J* = 6.6 Hz, 2H), 1.97 (t, *J* = 8.6 Hz, 2H), 1.60 (p, *J* = 6.7 Hz, 2H), 1.52 (ddd, *J* = 16.3, 8.5, 7.4 Hz, 2H), 1.40-1.27 (m, 4H); ¹³C NMR (101 MHz, DMSO-*d₆*) *δ* 169.80, 160.98, 156.66, 152.37, 138.67, 124.59, 124.11, 122.41, 121.85, 112.05, 111.02, 54.50, 34.80, 31.22, 26.86, 26.42, 25.11, 22.78.

### Example 18

(Z)-N-hydroxy-6-(4-(2-methoxy-4-(trifluoromethyl)benzylene)-2.5-dioxoimidazolidin-1-yl)hexanamide, with a structural formula as below: 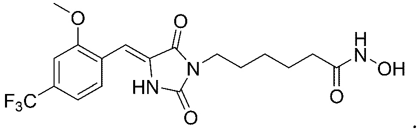 The starting material 2-methoxybenzaldehyde in Step 1 of Example 1 was replaced with 2-methoxy-4-trifluoromethylbenzaldehyde, and the fragment ethyl 7-bromoheptanoate in Step 2 was replaced with ethyl 6-bromohexanoate, while other steps and procedures were identical to those in Example 1. Light yellow solid, yield: 43%. ¹H NMR (400MHz, DMSO-*d₆*) *δ* 10.54 (d, *J* = 3.5 Hz, 1H), 9.00 (d, *J* = 3.5 Hz, 1H), 8.52 (s, 1H), 7.60 (d, *J* = 1.5 Hz, 2H), 7.31-7.27 (m, 1H), 6.82 (s, 1H), 3.98 (s, 2H), 3.75 (t, *J* = 6.6 Hz, 2H), 2.02 (t, *J* = 8.6 Hz, 2H), 1.69-1.54 (m, 4H), 1.44-1.35 (m, 2H); ¹³C NMR (101 MHz, DMSO-*d₆*) *δ* 169.40, 163.98, 158.10, 158.08, 158.06, 158.05, 154.37, 132.84, 132.59, 132.33, 132.07, 129.17, 129.15, 129.14, 129.12, 127.11, 127.04, 124.89, 124.39, 122.75, 120.60, 120.52, 120.49, 120.46, 120.42, 112.07, 110.64, 110.61, 110.58, 110.55, 56.07, 37.80, 32.22, 27.01, 26.28, 25.11.

### Example 19

(Z)-N-hydroxy-6-(2,5-dioxo-4-(3,4,5-trimethoxybenzylene)imidazolidin-1-yl)hexanamide, with a structural formula as below: 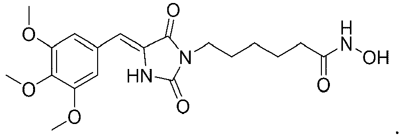 The starting material 2-methoxybenzaldehyde in Step 1 of Example 1 was replaced with 3,4,5-trimethoxybenzaldehyde, and the fragment ethyl 7-bromoheptanoate in Step 2 was replaced with ethyl 6-bromohexanoate, while other steps and procedures were identical to those in Example 1. White solid, yield: 34%. ¹H NMR (400 MHz, DMSO-*d₆*) *δ* 10.57 (d, *J* = 3.3 Hz, 1H), 9.02 (d, *J* = 3.5 Hz, 1H), 8.72 (s, 1H), 7.23 (s, 1H), 6.84 (s, 2H), 3.86 (s, 5H), 3.90 (s, 2H), 3.72 (t, *J* = 6.6 Hz, 2H), 1.97 (t, *J* = 8.6 Hz, 2H), 1.75-1.64 (m, 4H), 1.48-1.37 (m, 2H); ¹³C NMR (101 MHz, DMSO-*d₆*) *δ* 169.10, 162.70, 155.28, 154.84, 142.12, 131.78, 126.24, 114.45, 109.54, 62.74, 57.42, 36.90, 33.81, 27.87, 27.22, 25.04.

### Example 20

(Z)-N-hydroxy-6-(4-(naphth-1-ylmethylene)-2,5-dioxoimidazolidin-1-yl)hexanamide, with a structural formula as below: 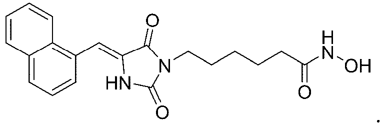 The starting material 2-methoxybenzaldehyde in Step 1 of Example 1 was replaced with 1-naphthaldehyde, and the fragment ethyl 7-bromoheptanoate in Step 2 was replaced with ethyl 6-bromohexanoate, while other steps and procedures were identical to those in Example 1. Light yellow solid, yield: 37%. ¹H NMR (400 MHz, DMSO-*d₆*) *δ* 10.85 (d, *J* = 3.3 Hz, 1H), 9.10 (d, *J* = 3.5 Hz, 1H), 8.87 (s, 1H), 8.10-8.02 (m, 1H), 7.94-7.87 (m, 2H), 7.61 (t, *J* = 7.7 Hz, 1H), 7.60-7.52 (m, 2H), 7.48 (d, *J* = 7.8 Hz, 1H), 6.92 (s, 1H), 3.72 (t, *J* = 6.6 Hz, 2H), 2.00 (t, *J* = 8.6 Hz, 2H), 1.70-1.55 (m, 4H), 1.45-1.36 (m, 2H); ¹³C NMR (101 MHz, DMSO-*d₆*) *δ* 169.87, 161.15, 154.38, 133.08, 132.74, 132.36, 129.24, 128.34, 128.30, 128.25, 126.96, 126.74, 125.72, 124.74, 110.87, 37.80, 32.22, 28.01, 27.29, 25.31.

### Example 21

(Z)-N-hydroxy-6-(4-(naphth-2-ylmethylene)-2,5-dioxoimidazolidin-1-yl)hexanamide, with a structural formula as below: 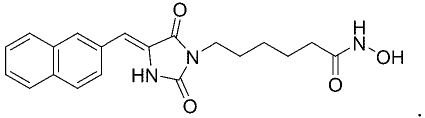 The starting material 2-methoxybenzaldehyde in Step 1 of Example 1 was replaced with 2-naphthaldehyde, and the fragment ethyl 7-bromoheptanoate in Step 2 was replaced with ethyl 6-bromohexanoate, while other steps and procedures were identical to those in Example 1. Light yellow solid, yield: 35%. ¹H NMR (400 MHz, DMSO-*d₆*) *δ* 10.58 (d, *J* = 3.3 Hz, 1H), 9.02 (d, *J* = 3.5 Hz, 1H), 8.89 (s, 1H), 7.98 (dt, *J* = 6.8, 2.0 Hz, 1H), 7.87 (ddd, *J* = 5.7, 2.9, 1.2 Hz, 2H), 7.72 (d, *J* = 8.2 Hz, 1H), 7.58-7.49 (m, 3H), 6.52 (s, 1H), 3.78 (t, *J* = 6.6 Hz, 2H), 1.97 (t, *J* = 8.6 Hz, 2H), 1.69-1.53 (m, 4H), 1.41-1.30 (m, 2H); ¹³C NMR (101 MHz, DMSO-*d₆*) *δ* 170.80, 166.09, 152.38, 135.52, 135.00, 132.05, 130.06, 129.11, 128.84, 128.28, 127, 86, 127.74, 127.64, 118.25, 36.90, 34.35, 26.81, 26.08, 24.81.

### Example 22

(Z)-N-hydroxy-6-(2.5-dioxo-4-(quinolin-2-ylmethylene)imidazolidin-1-yl)hexanamide, with a structural formula as below: 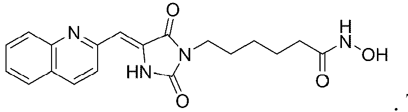 The starting material 2-methoxybenzaldehyde in Step 1 of Example 1 was replaced with 2-quinolinecarboxaldehyde, and the fragment ethyl 7-bromoheptanoate in Step 2 was replaced with ethyl 6-bromohexanoate, while other steps and procedures were identical to those in Example 1. Light yellow solid, yield: 31%. ¹H NMR (400 MHz, DMSO-*d₆*) *δ* 10.76 (d, *J* = 3.3 Hz, 1H), 9.15 (d, *J* = 3.5 Hz, 1H), 8.74 (s, 1H), 8.27 (d, *J* = 8.1 Hz, 1H), 8.09-7.99 (m, 1H), 7.95-7.88 (m, 1H), 7.75-7.69 (m, 2H), 7.58 (s, 1H), 7.51 (td, *J* = 7.9, 1.3 Hz, 1H), 3.78 (t, *J* = 6.6 Hz, 2H), 1.98 (t, *J* = 8.6 Hz, 2H), 1.70-1.54 (m, 4H), 1.46-1.32 (m, 2H); ¹³C NMR (101 MHz, DMSO-*d₆*) *δ* 169.89, 164.14, 154.31, 149.81, 147.93, 136.17, 129.80, 128.76, 128.56, 128.53, 126.98, 126.85, 122.01, 108.77, 37.80, 32.22, 28.01, 27.26, 26.86, 25.01.

### Example 23

(Z)-N-hydroxy-6-(2.5-dioxo-4-(quinolin-3-ylmethylene)imidazolidin-1-yl)hexanamide, with a structural formula as below: 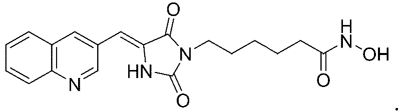 The starting material 2-methoxybenzaldehyde in Step 1 of Example 1 was replaced with 3-quinolinecarboxaldehyde, and the fragment ethyl 7-bromoheptanoate in Step 2 was replaced with ethyl 6-bromohexanoate, while other steps and procedures were identical to those in Example 1. Light yellow solid, yield: 32%. ¹H NMR (400 MHz, DMSO-*d₆*) *δ* 10.65 (d, *J* = 3.3 Hz, 1H), 9.12 (d, *J* = 3.5 Hz, 1H), 8.92 (s, 1H), 8.80 (d, *J* = 2.0 Hz, 1H), 8.38 (t, *J* = 1.9 Hz, 1H), 8.10 (dt, *J* = 8.2, 1.8 Hz, 1H), 8.02 (dd, *J* = 7.2, 1.3 Hz, 1H), 7.68 (td, *J* = 7.5, 1.3 Hz, 1H), 7.62 (td, *J* = 8.2.1.4 Hz, 1H), 6.87 (s, 1H), 3.84 (t, *J* = 6.6 Hz, 2H), 2.00 (t, *J* = 8.6 Hz, 2H), 1.65-1.48 (m, 4H),1.40-1.30 (m, 2H); ¹³C NMR (101 MHz, DMSO-*d₆*) *δ* 171.80, 166.12, 151.38, 148.02, 147.33, 132.67, 128.95, 128.65, 128.56, 128.43, 127.44, 126.81, 126.60, 113.70, 38.87, 33.54, 27.87, 27.27, 25.31.

### Example 24

(Z)-N-hydroxy-6-(2.5-dioxo-4-(quinolin-4-ylmethylene)imidazolidin-1-yl)hexanamide, with a structural formula as below: 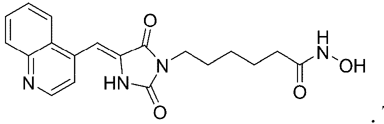 The starting material 2-methoxybenzaldehyde in Step 1 of Example 1 was replaced with 4-quinolinecarboxaldehyde, and the fragment ethyl 7-bromoheptanoate in Step 2 was replaced with ethyl 6-bromohexanoate, while other steps and procedures were identical to those in Example 1. Light yellow solid, yield: 30%. ¹H NMR (400 MHz, DMSO-*d₆*) *δ* 10.48 (d, *J* = 3.3 Hz, 1H), 8.92 (d, *J* = 3.5 Hz, 1H), 8.70 (s, 1H), 8.71 (d, *J* = 4.9 Hz, 1H), 8.27-8.15 (m, 1H), 7.95 (dd, *J* = 8.2, 1.5 Hz, 1H), 7.61 (ddd, *J* = 8.0, 6.9, 1.2 Hz, 1H), 7.52 (ddd, *J* = 8.2, 7.0, 1.3 Hz, 1H), 7.40 (d, *J* = 4.9 Hz, 1H), 6.89 (s, 1H), 3.72 (t, *J* = 6.6 Hz, 2H), 1.97 (t, *J* = 8.6 Hz, 2H), 1.60-1.45 (m, 4H), 1.40-1.29 (m, 2H); ¹³C NMR (101 MHz, DMSO-*d₆*) *δ* 170.80, 162.16, 154.38, 148.08, 148.03, 136.58, 129.92, 129.80, 129.23, 127.88, 127.80, 124.29, 122.99, 111.37, 38.10, 34.55, 27.08, 26.84, 25.01.

### Example 25

(Z)-N-hydroxy-6-(2.5-dioxo-4-(quinolin-6-ylmethylene)imidazolidin-1-yl)hexanamide, with a structural formula as below: 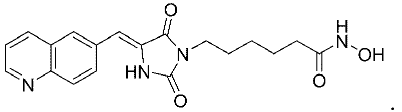 The starting material 2-methoxybenzaldehyde in Step 1 of Example 1 was replaced with 6-quinolinecarboxaldehyde, and the fragment ethyl 7-bromoheptanoate in Step 2 was replaced with ethyl 6-bromohexanoate, while other steps and procedures were identical to those in Example 1. Light yellow solid, yield: 32%. ¹H NMR (400 MHz, DMSO-*d₆*) *δ* 10.54 6*J* = 3.3 Hz, 1H), 9.02 (d, *J* = 3.5 Hz, 1H), 8.95 (s, 1H), 8.89 (dd, *J* = 4.1, 1.7 Hz, 1H), 8.26 (dt, *J* = 8.4, 1.8 Hz, 1H), 8.15-8.09 (m, 1H), 8.01 (d, *J* = 8.2 Hz, 1H), 7.68 (dd, *J* = 8.2, 2.2 Hz, 1H), 7.55 (dd, *J* = 8.2, 4.1 Hz, 1H), 6.65 (s, 1H), 3.70 (t, *J* = 6.6 Hz, 2H), 1.98 (t, *J* = 8.6 Hz, 2H), 1.69-1.52 (m, 4H), 1.41-1.30 (m, 2H); ¹³C NMR (101 MHz, DMSO-*d₆*) *δ* 169.24, 162.09, 155.38, 148.65, 145.76, 131.38, 130.17, 129.05, 128.37, 127.83, 127.28, 127.27, 122.01, 116.60, 39.40, 34.51, 26.98, 26.64, 25.54.

### Example 26

(Z)-N-hydroxy-6-(2,5-dioxo-4-(quinolin-5-ylmethylene)imidazolidin-1-yl)hexanamide, with a structural formula as below: 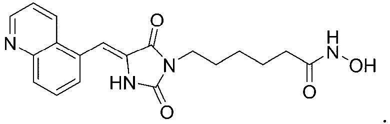 The starting material 2-methoxybenzaldehyde in Step 1 of Example 1 was replaced with 8-quinolinecarboxaldehyde, and the fragment ethyl 7-bromoheptanoate in Step 2 was replaced with ethyl 6-bromohexanoate, while other steps and procedures were identical to those in Example 1. Light yellow solid, yield: 30%. ¹H NMR (400 MHz, DMSO-*d₆*) *δ* 110.56 (d, *J* = 3.3 Hz, 1H), 9.13 (d, *J* = 3.5 Hz, 1H), 8.95 (dd, *J* = 4.2, 1.8 Hz, 1H), 8.72 (s, 1H), 8.39 (dd, *J* = 7.6, 1.8 Hz, 1H), 8.01 (dd, *J* = 7.2, 1.3 Hz, 1H), 7.65 (dd, *J* = 8.3, 7.2 Hz, 1H), 7.57-7.45 (m, 1H), 7.43 (dd, *J* = 7.8, 4.1 Hz, 1H), 7.02 (s, 1H), 3.72 (t, *J* = 6.6 Hz, 2H), 1.97 (t, *J* = 8.6 Hz, 2H), 1.66-1.50 (m, 4H), 1.45-1.31 (m, 2H); ¹³C NMR (101 MHz, DMSO-*d₆*) *δ* 171.01, 163.11, 152.12, 145.24, 144.53, 131.05, 130.12, 129.26, 129.03, 128.51, 128.44, 127.89, 123.40, 111.55, 37.80, 32.22, 27.01 , 26.28, 24.41.

### Example 27

(Z)-N-hydroxy-6-(4-(furan-2-ylmethylene)-2.5-dioxoimidazolidin-1-yl)hexanamide, with a structural formula as below: 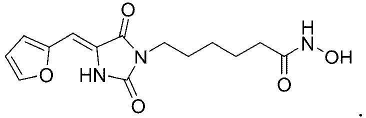 The starting material 2-methoxybenzaldehyde in Step 1 of Example 1 was replaced with 2-furaldehyde, and the fragment ethyl 7-bromoheptanoate in Step 2 was replaced with ethyl 6-bromohexanoate, while other steps and procedures were identical to those in Example 1. Dark solid, yield: 31%. ¹H NMR (400 MHz, DMSO-*d₆*) *δ* 10.66 (d, *J* = 3.3 Hz, 1H), 9.06 (d, *J* = 3.5 Hz, 1H), 8.75 (s, 1H), 7.89 (t, *J* = 1.5 Hz, 1H), 6.96 (dd, *J* = 5.0, 1.2 Hz, 1H), 6.76 (s, 1H), 6.58 (dd, *J* = 5.0, 1.7 Hz, 1H), 3.70 (t, *J* = 6.6 Hz, 2H), 1.99 (t, *J* = 8.6 Hz, 2H), 1.66-1.55 (m, 4H), 1.45-1.31 (m, 2H); ¹³C NMR (101 MHz, DMSO-*d₆*) *δ* 172.08, 160.34, 153.38, 151.83, 145.11, 127.00, 115.71, 111.59, 99.81, 37.80, 32.22, 27.01, 26.28, 25.14.

### Example 28

### (Z)-N-hydroxy-6-(2.5-dioxo-4-(thiophen-2-ylmethylene)imidazolidin-1-yl)hexanamide:

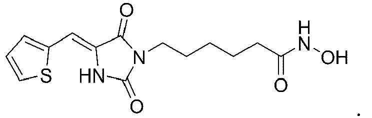

The starting material 2-methoxybenzaldehyde in Step 1 of Example 1 was replaced with 2-thiophenecarboxaldehyde, and the fragment ethyl 7-bromoheptanoate in Step 2 was replaced with ethyl 6-bromohexanoate, while other steps and procedures were identical to those in Example 1. Yellow solid, yield: 35%. ¹H NMR (400 MHz, DMSO-*d₆*) *δ* 10.60 (d, *J* = 3.5 Hz, 1H), 9.02 (d, *J* = 3.5 Hz, 1H), 8.55 (s, 1H), 7.61 (dd, *J* = 7.7, 1.6 Hz, 1H), 7.35 (td, *J* = 7.8, 1.6 Hz, 1H), 7.02 (td, *J* = 7.6, 1.2 Hz, 1H), 6.98 (dd, *J* = 8.0, 1.3 Hz, 1H), 6.84 (s, 1H), 3.85 (s, 2H), 3.70 (t, *J* = 6.6 Hz, 2H), 1.92 (t, *J* = 8.6 Hz, 2H), 1.61 (p, *J* = 6.7 Hz, 2H), 1.54 (ddd, *J* = 16.3, 8.5, 7.4 Hz, 2H), 1.40-1.28 (m, 4H); ¹³C NMR (101 MHz, DMSO-*d₆*) *δ* 170.80, 162.50, 154.37, 137.94, 130.79, 128.83, 126.40, 124.32, 107.97, 37.80, 34.22, 26.51, 26.18, 25.51.

### Example 29

### (Z)-N-hydroxy-6-(4-((2,3-dihydrobenzofuran-5-yl)methylene)-2,5-dioxoimidazolidin-1-yl)hexanamide:

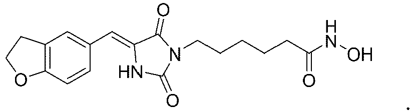

The starting material 2-methoxybenzaldehyde in Step 1 of Example 1 was replaced with 2,3-dihydrobenzofuran-5-carbaldehyde, and the fragment ethyl 7-bromoheptanoate in Step 2 was replaced with ethyl 6-bromohexanoate, while other steps and procedures were identical to those in Example 1. White solid, yield: 28%. ¹H NMR (400 MHz, DMSO-*d₆*) *δ* 10.61 (d, *J* = 3.3 Hz, 1H), 9.08 (d, *J* = 3.5 Hz, 1H), 8.88 (s, 1H), 7.40 (dt, *J* = 2.4, 0.9 Hz, 1H), 7.39 (dd, *J* = 8.9, 2.3 Hz, 1H), 7.05 (s, 1H), 6.94 (d, *J* = 8.9 Hz, 1H), 4.52 (ddd, *J* = 13.7, 5.1, 3.3 Hz, 2H), 3.75 (t, *J* = 6.6 Hz, 2H), 3.21 (ddd, *J* = 5.1, 3.3, 1.0 Hz, 1H), 3.15 (ddd, *J* = 5.1, 3.2, 1.0 Hz, 1H), 2.02 (t, *J* = 8.6 Hz, 2H), 1.70-1.56 (m, 4H), 1.41-1.32 (m, 2H); ¹³C NMR (101 MHz, DMSO-*d₆*) *δ* 170.22, 164.09, 160.24, 154.38, 130.17, 128.83, 127.44, 126.41, 125.77, 116.42, 110.54, 71.81, 38.78, 35.88, 29.73, 26.85, 26.28, 25.41.

### Example 30

(Z)-N-hydroxy-6-(4-(benzofuran-5-ylmethylene)-2.5-dioxoimidazolidin-1-yl)hexanamide, with a structural formula as below: 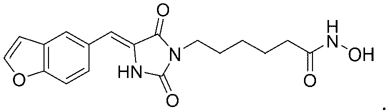 The starting material 2-methoxybenzaldehyde in Step 1 of Example 1 was replaced with 1-benzofuran-5-carbaldehyde, and the fragment ethyl 7-bromoheptanoate in Step 2 was replaced with ethyl 6-bromohexanoate, while other steps and procedures were identical to those in Example 1. White solid, yield: 24%. ¹H NMR (400 MHz, DMSO-*d₆*) *δ* 110.56 (d, *J =* 3.3 Hz, 1H), 9.06 (d, *J =* 3.51z.1H), 8.86 (s, 1H), 8.05 (d, *J=* 1.6 Hz, 1H), 7.96-7.88 (m, 1H), 7.65 (d, *J=* 8.4 Hz, 1H), 7.60 (dd, *J=* 8.4, 2.2 Hz, 1H), 6.81 (t, *J =* 1.8 Hz, 1H), 6.62 (s, 1H), 3.72 (t, *J =* 6.6 Hz, 2H), 1.96 (t, *J* = 8.6 Hz, 2H), 1.60-1.45 (m, 4H), 1.40-1.32 (m, 2H); ¹³C NMR (101 MHz, DMSO-*d₆*) *δ* 169.95, 163.09, 155.98, 154.38, 145.74, 130.79, 128.96, 127.56, 125.84, 123.47, 117.25, 112.37, 106.95, 37.80, 35.85, 26.62, 26.22, 25.38.

### Example 31

(Z)-N-hydroxy-6-(4-(benzo[[1,3]dioxol-5-ylmethylene)-2,5-dioxoimidazolidin-1-yl)hexanamide, with a structural formula as below: 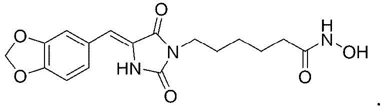 The starting material 2-methoxybenzaldehyde in Step 1 of Example 1 was replaced with 3,4-methylenedioxybenzaldehyde (piperonal), and the fragment ethyl 7-bromoheptanoate in Step 2 was replaced with ethyl 6-bromohexanoate, while other steps and procedures were identical to those in Example 1. White solid, yield: 40%. ¹H NMR (400 MHz, DMSO-*d₆*) *δ* 10.53 (d, *J =* 3.3 Hz, 1H), 9.01 (d, *J* = 3.5 Hz, 1H), 8.77 (s, 1H), 7.12 (d, *J* = 2.0 Hz, 1H), 7.09 (dd, *J =* 8.8, 2.0 Hz, 1H), 6.96 (d, *J =* 8.8 Hz, 1H), 6.66 (s, 1H), 6.00 (s, 2H), 3.75 (t, *J =* 6.6 Hz, 2H), 1.99 (t, *J =* 8.6 Hz, 2H), 1.69-1.50 (m, 4H), 1.41-1.32 (m, 2H); ¹³C NMR (101 MHz, DMSO-*d₆*) *δ* 170.12, 164.09, 154.38, 150.14, 147.86, 128.85, 125.62, 124.45, 115.35, 110.08, 109.64, 102.28, 37.80, 35.52, 26.70, 26.20, 25.52.

### Example 32

(Z)-N-hydroxy-6-(4-((2.2-difluorobenzo[d][1.3]dioxol-5-yl)methylene)-2.5-dioxoimidazolidin-1-yl)hexanamide, with a structural formula as below: 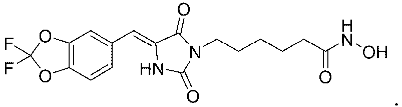 The starting material 2-methoxybenzaldehyde in Step 1 of Example 1 was replaced with 2,2-difluoro-1,3-benzodioxole-5-carbaldehyde, and the fragment ethyl 7-bromoheptanoate in Step 2 was replaced with ethyl 6-bromohexanoate, while other steps and procedures were identical to those in Example 1. Light yellow solid, yield: 33%. ¹H NMR (400 MHz, DMSO-*d₆*) *δ* 10.57 (d, *J =* 3.3 Hz, 1H), 9.11 (d, *J* = 3.5 Hz, 1H), 8.78 (s, 1H), 7.24 (d, *J =* 2.0 Hz, 1H), 7.19 (d, *J =* 8.1 Hz, 1H), 7.11 (dd, *J =* 8.0.1.9 Hz, 1H), 6.66 (s, 1H), 3.72 (t, *J =* 6.6 Hz, 2H), 1.99 (t, *J =* 8.6 Hz, 2H), 1.67-1.52 (m, 4H), 1.42-1.33 (m, 2H); ¹³C NMR (101 MHz, DMSO-*d₆*) *δ* 169.86, 164.09, 154.38, 143.72, 143.63, 143.55, 142.72, 142.63, 142.55, 134.27, 132.13, 129.98, 128.97, 125.62, 124.36, 115.49, 113.94, 113.93, 113.69, 38.80, 35.22, 26.77, 26.58, 25.01.

### Example 33

(Z)-N-(2-amino-4-fluorophenyl)-4-((2.5-dioxo-4-(pyridin-3-ylmethylene)imidazolidin-1-yl)methyl)benzamide, with a structural formula as below: 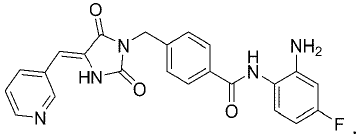 The starting material 2-methoxybenzaldehyde in Step 1 of Example 1 was replaced with 3-pyridinecarbaldehyde, the fragment ethyl 7-bromoheptanoate in Step 2 was replaced with methyl 4-bromomethylbenzoate, and the starting material hydroxylamine hydrochloride in Step 4 was replaced with 4-fluoro-1,2-phenylenediamine, while other steps and procedures were identical to those in Example 1. Light yellow solid, yield: 30%. ¹H NMR (400 MHz, DMSO-*d₆*) *δ* 9.45 (s, 1H), 8.89 (s, 1H), 8.73-8.69 (m, 1H), 8.53 (ddd, *J =* 3.9.1.9.1.1 Hz, 1H), 7.96-7.90 (m, 2H), 7.74 (dt, *J* = 7.7, 1.9 Hz, 1H), 7.67 (dd, *J =* 8.2, 5.0 Hz, 1H), 7.41 (dt, *J =* 8.4, 1.0 Hz, 2H), 7.33 (dd, *J =* 7.8, 3.8 Hz, 1H), 6.86-6.76 (m, 2H), 6.77 (s, 1H), 4.87-4.82 (m, 4H); ¹³C NMR (101 MHz, DMSO-*d₆*) *δ* 173.88, 163.71, 159.89, 157.87, 153.97, 150.39, 149.90, 142.01, 141.95, 137.31, 134.82, 132.71, 130.72, 129.00, 128.44, 126.22, 126.20, 125.90, 123.64, 122.86, 122.80, 114.39, 106.69, 106.53, 102.71, 102.55, 39.76.

### Example 34

(Z)-N-(2-amino-4-fluorophenyl)-4-((2,5-dioxo-4-(pyridin-3-ylmethylene)imidazolidin-1-yl)methyl)benzamide, with a structural formula as below:

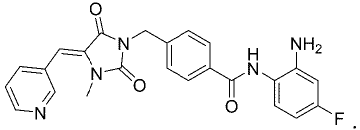

The starting material 2-methoxybenzaldehyde in Step 1 of Example 1 was replaced with 3-pyridinecarbaldehyde, hydantoin in Step 1 was replaced with 1-methylhydantoin, the fragment ethyl 7-bromoheptanoate in Step 2 was replaced with methyl 4-bromomethylbenzoate, and the starting material hydroxylamine hydrochloride in Step 4 was replaced with 4-fluoro-1,2-phenylenediamine, while other steps and procedures were identical to those in Example 1. Light yellow solid, yield: 31%. ¹H NMR (400 MHz, DMSO-*d₆*) *δ* 9.45 (s, 1H), 8.77-8.73 (m, 1H), 8.53 (ddd, *J* = 3.8, 1.9, 1.1 Hz, 1H), 7.96-7.90 (m, 2H), 7.82 (dt, *J =* 7.9, 1.9 Hz, 1H), 7.67 (dd, *J =* 8.2, 5.0 Hz, 1H), 7.41 (dt, *J* = 8.5, .0 Hz, 2H), 7.33 (dd, *J* = 7.8, 3.9 Hz, 1H), 7.00 (s, 1H), 6.83 (td, *J* = 8.1.2.2 Hz, 1H), 6.78 (dd, *J* = 8.0, 2.3 Hz, 1H), 4.93 (t, *J* = 1.0 Hz, 2H), 4.84 (s, 2H), 3.30 (s, 2H); ¹³C NMR (101 MHz, DMSO-*d₆*) *δ* 173.88, 160.56, 159.89, 157.87, 154.94, 149.90, 149.87, 142.01, 141.95, 138.04, 134.62, 133.21, 132.71, 132.52, 129.00, 128.47, 126.22, 126.20, 123.63, 122.86, 122.80, 116.70, 106.69, 106.53, 102.71, 102.55, 41.30, 29.11.

### Example 35

Synthesis of (Z)-N-hydroxy-6-(2,5-dioxo-4-(4-(pyridin-4-yl)quinolin-6-yl)methylene)imidazolidin-1-yl)hexanamide, with synthesis steps as below.

Step 1: Synthesis of 4-(pyridin-4-yl)quinolin-6-formaldehyde, with a structural formula as below: 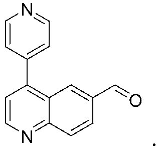 30 mmol cycl-isopropylidene malonate (1.5 eq.) was dissolved in 20 mL of trimethyl orthoformate and then added to a 100 mL three-necked flask for nitrogen displacement, with the reaction temperature raised to 110 °C, allowing a reaction to proceed for 2 h under reflux; then, 20 mmol of methyl 4-aminobenzoate was weighed and dissolved in 20 mL of trimethyl orthoformate and then carefully added to the reaction flask for a further reaction to proceed for 2 h; then the reaction mixture was cooled to room temperature, concentrated to remove a solvent via evaporation , washed with methanol, and filtered to obtain white solids; the white solids were dried, added to 40 mL of diphenyl ether, and subjected to nitrogen displacement for 10-20 min with the reaction temperature raised to 240 °C; after cooling, 20 mL of n-hexane was added; the reaction mixture was further stirred and filtered to obtain solids; and the solids were washed with n-hexane and dried to obtain a compound G (methyl 4-oxo-4,4a-dihydroquinolin-6-carboxylate, yield: 78%) as light yellow solid. 3.3 mmol compound G was dissolved in 10 mL of DMF, and 341 µL of phosphorus tribromide (1.1 eq.) was slowly added dropwise at room temperature, allow a reaction to proceed for 1 h under the protection of nitrogen gas. Upon completion of the reaction, the reaction mixture was slowly poured into 100 mL of ice-cold water (with vigorous stirring throughout); sodium acetate was added (to neutralize the reaction products of phosphorus tribromide and water; the mixture was then extracted with an ethyl acetate solution (3 × 60 mL); organic phases were collected, washed with saturated saline (2 × 200 mL), dried over anhydrous magnesium sulfate, and purified by column chromatography (PE:EA = 3:1) to obtain a light yellow compound H (methyl 4-bromoquinolin-6-carboxylate, yield: 88%). 2 mmol compound H, 3 mmol 4-pyridinylboronic acid, 0.1 mmol Pd(dppf)Cl₂ (5 mol%) and 4 mmol potassium carbonate were added to a reaction flask containing 15 mL of 1,4-dioxane and 3 mL of water, allowing a reaction to proceed for 2 h at a reaction temperature of 80 °C under a nitrogen atmosphere; then, the reaction mixture was cooled to room temperature and filtered to remove solids; and a residue was purified by column chromatography (PE:EA = 1:1) to obtain a light yellow compound I (methyl 4-(pyridin-4-yl)quinolin-6-carboxylate, yield: 90%). 1 mmol compound I was added to 4 mL of anhydrous THF under the protection of nitrogen gas; at 0 °C, 1.6 mL of a solution of LiAlH₄ in THF (2.5mol/L, 4.0 eq.) was slowly added; the reaction temperature was raised to room temperature; after 2 h, 0.16 g of water was added to quench the reaction; the reaction mixture was stirred for 10 min, followed by the sequential addition of 0.16 g of 10% NaOH in water and 0.48 g of water; a resulting mixture was dried over anhydrous magnesium sulfate and filtered to remove solids; and a residue was concentrated to obtain a crude light yellow compound J ((4-(pyridin-4-yl)quinolin-6-yl)methanol). 1 mmol crude compound J was dissolved in 10 mL of DCM; under room-temperature conditions, 1.2 mmol DMP (dissolved in 10 mL of DCM) was slowly added dropwise, allowing a reaction to proceed for 5 h; 20 mL of a DCM-diluted reaction mixture was added; a resulting mixture was sequentially washed with a saturated aqueous sodium carbonate solution (40 mL), a saturated sodium thiosulfate solution (40 mL) and saturated saline (2 × 40mL), dried over anhydrous magnesium sulfate, and purified by column chromatography (DCM:MeOH = 20:1) to obtain a light yellow compound K (4-(pyridin-4-yl) quinolin-6-formaldehyde, yield: 83%). ¹H NMR (400 MHz, Chloroform-*d*) *δ* 8.71-8.64(m,3H), 8.52(d, *J* = 4.8 Hz, 1H), 8.10(d, *J* = 8.2 Hz, 1H), 8.03 (dd, *J* = 8.1,2.2 Hz, 1H), 7.88-7.83(m,2H), 7.80(d, *J* = 4.6 Hz, 1H).

Step 2: Synthesis of (Z)-N-hydroxy-6-(2,5-dioxo-4-((4-(pyridin-4-yl)quinolin-6-yl)methylene)imidazolidin-1-yl)hexanamide, with a structural formula as below: 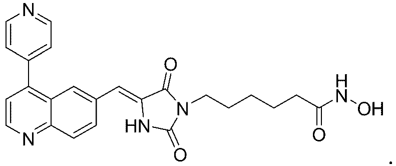 The starting material 2-methoxybenzaldehyde in Step 1 of Example 1 was replaced with 4-(pyridin-4-yl)quinolin-6-carbaldehyde, and the fragment ethyl 7-bromoheptanoate in Step 2 was replaced with ethyl 6-bromohexanoate, while other steps and procedures were identical to those in Example 1, yield: 40%. ¹H NMR (400 MHz, DMSO-*d₆*) *δ* 10.53 (d, *J* = 3.3 Hz, 1H), 9.03 (d, *J* = 3.5 Hz, 1H), 8.84 (s, 1H), 8.69-8.64 (m, 2H), 8.48 (d, *J* = 4.8 Hz, 1H). 8.33 (d, *J* = 2.6 Hz, 1H), 8.03 (d, *J* = 8.2 Hz, 1H), 7.88-7.83 (m, 2H), 7.75 (d, *J* = 4.7 Hz, 1H), 7.61 (dd, *J* = 8.2, 2.2 Hz, 1H), 6.68 (s, 1H), 3.72 (t, *J* = 6.6 Hz, 2H), 2.01 (t, *J* = 8.6 Hz, 2H), 1.68-1.53 (m, 4H), 1.41-1.30 (m, 2H); ¹³C NMR (101 MHz, DMSO-*d₆*) *δ* 169.53, 164.09, 154.38, 149.88, 149.55, 146.53, 141.95, 139.80, 132.11, 128.92, 128.87, 127.25, 126.96, 123.87, 122.06, 121.54, 115.90, 36.84, 35.10, 26.45, 26.51, 25.01.

HDAC inhibition assays were conducted for the inhibitors of the present invention:
ADP-Glo Luminescent Kinase Assay was used for testing, and reagents used in the kinase reaction were as follows: HEPES (50 mM) pH 7.5 with NaCl (100 mM), EGTA(1.0 mM), MgCl₂(3.0 mM), DTT (2.0 mM) and CHAPS (0.03%). During the reaction, 50 µM PIP2 and 25 µM ATP were added to 10 mL of each test compound at varying concentrations (0.05 nM to 1.0 µM). A reaction mixture was incubated at room temperature for 1 h, and then, 10 µL of an ADP-Glo reagent was added to terminate an enzymatic reaction. Data were collected using Envision software, and the IC₅₀ values of the compounds were analyzed and fitted using Graphpad Prism 5.

**Table 1: HDAC1 inhibitory activity (IC₅₀, nM) of compounds of some examples**

| Example | Example 1 | Example 2 | Example 3 | Example 4 | Example |
|---|---|---|---|---|---|
| HDAC1 | 8.6 | 13 | 2.8 | 76 | 6.4 |

| Example | Example 6 | Example 7 | Example 8 | Example 9 | Example 1 |
|---|---|---|---|---|---|
| HDAC1 | 7.8 | 25 | 68 | 16 | 6 |

| Example | Example 11 | Example 12 | Example 13 | Example 14 | Example 19 |
|---|---|---|---|---|---|
| HDAC1 | 0.57 | 1.7 | 1.0 | 1.2 | 1.2 |

| Example | Example 2 | Example 21 | Example 23 | Example 31 | Example 33 |
|---|---|---|---|---|---|
| HDAC1 | 3.6 | 2.4 | 2.0 | 5.9 | 180 |

| Example | Example 34 | Vorinostat | Tucidinostat | | |
|---|---|---|---|---|---|
| HDAC1 | 175 | 15.0 | 122.1 | | |

As shown in Table 1, the compounds of the present invention exhibit a nanomolar-level inhibitory activity against HDAC1, and some of the compounds are significantly superior to the positive control Vorinostat. In particular, Example 11 exhibits that its inhibitory activity against HDAC1 is nearly 30 times that of the positive control Vorinostat. This indicates that the compounds in the examples of the present invention are efficient HDAC inhibitors.

Anti-proliferative activity assays in tumor cell lines were conducted for the inhibitors of the present invention.
The inhibitory activity of the compounds against cell proliferation was evaluated using the CCK 8 assay, and initial single-concentration screening and multi-concentration assays were conducted to determine the half-maximal inhibitory concentration (IC₅₀) values. Detection principle: the cytotoxicity (CCK 8 assay) detection principle was as follows: a CCK 8 reagent contained WST-8, which was reduced by dehydrogenases in cell mitochondria under the action of an electron carrier 1-methoxy-5-methylphenazine dimethyl sulfate (1-Methoxy PMS) to produce a highly water-soluble yellow formazan product. The amount of the produced formazan product was directly proportional to the number of live cells.

Experimental methods were as follows.
(1) Cell seeding: a single-cell suspension was prepared using a culture medium containing 10% fetal bovine serum; 90 µL of adherent cells (5 × 10⁴/mL) and suspension cells (9 × 10⁴/mL) was seeded into each well of a 96-well plate; and the cells were pre-incubated for 24 h at 37°C in the presence of 5% CO₂.
(2) Addition of test sample solution: 10 µL of a sample solution was added to each well; for initial activity screening, one concentrate was set per sample, with three replicates; for IC₅₀ determination, eight concentrations (including the concentration of 0) were used, with three replicates set per concentration; and the incubation was conducted for 48 h in an incubator. In this experiment, a blank group (Blank), a control group (Control) and a drug group (Drug) were provided.
(3) Color development: For the adherent cells, the old culture medium and the drug solution were aspirated (for suspension cells, they were directly added to 10 µL of undiluted CCK 8 solution); 100 µL of the CCK 8 solution diluted 10-fold was added to each well; and the incubation was continued for 14 h at 37°C in the presence of 5% CO₂ (with the procedures monitored in real time).
(4) Detection: The absorbance at 450 nm was measured using a microplate reader, and raw data results were recorded.
(5) The raw data were standardized using Excel software; the cell proliferation inhibition rate in each well during initial screening was calculated using the OD value (via the formula: inhibition rate = (OD _{Control}- OD _{Drug})/(OD _{Control}- OD _{Blank})×100%), and the statistical analysis of the inhibition rates was performed. IC₅₀ was calculated using GraphPad Prism 8.

**Table 2: Anti-proliferative activity (IC₅₀, µM) of some compounds of examples against tumor cell lines**

| Example | Example 1 | Example 2 | Example 3 | Example 4 | Example 5 | Example 6 | Example 7 |
|---|---|---|---|---|---|---|---|
| A549 | 5.81 | 9.70 | 7.94 | 6.82 | 5.03 | 7.60 | - |
| DUI45 | 0.69 | 1.45 | 0.26 | >4 | >4 | 1.65 | >4 |

| Example | Example 8 | Example 9 | Example 10 | Example 11 | Example 12 | Example 13 | Example 1 |
|---|---|---|---|---|---|---|---|
| A549 | | 4.43 | 5.13 | 2.36 | 9.74 | 1.49 | 2.02 |
| DU145 | >4 | 2.26 | 3.53 | <0.10 | <0.10 | <0.10 | <0.10 |

| Example | Example 15 | Example 16 | Example 17 | Example 18 | Example 20 | Example 21 | Example 23 |
|---|---|---|---|---|---|---|---|
| A549 | 4.86 | 2.42 | 2.04 | 2.12 | 4.30 | 3.68 | |
| DU145 | <0.10 | <0.10 | <0.10 | <0.10 | 1.28 | 0.77 | 3.43 |

| Example | Example 28 | Example 29 | Example 30 | Vorinostat | Tucidinostat | | |
|---|---|---|---|---|---|---|---|
| A549 | 4.86 | 3.68 | 4.43 | 1.62 | 9.85 | | |
| DU145 | 1.04 | <0.10 | 0.34 | 0.64 | 1.62 | | |

As can be seen from Table 2, the compounds of the present invention exhibit a micromolar-level anti-proliferative activity against two types of tumor cell lines, and in particular, the compounds of the examples show high sensitivity towards the human prostate cancer cell line DU-145. Here, nine compounds of Examples 11 to 18 and 29 all exhibit an inhibitory activity of lower than 100 nanomoles against DU-145, which is significantly superior to that of the positive control Vorinostat. As can be seen from this, the compounds in the examples of the present invention can be potentially applied to the clinical treatment of the tumors described above.

The imidazolidinedione-based HDAC inhibitor of the present invention is highly effective for inhibiting HDAC and the proliferation of solid tumor cells, and has anti-tumor efficacy and high sensitivity towards prostate cancer, showing potential for clinical tumor treatment.

In addition to the implementations described above, other embodiments are also possible for the present invention. Any technical solutions derived by equivalent substitution or transformation shall fall within the scope of protection of the present invention.

## Claims

1. An imidazolidinedione-based HDAC inhibitor, wherein the inhibitor is a substituted imidazolidinedione compound of Formula V below, or a stereisomer, hydrate or pharmaceutically acceptable salt thereof: 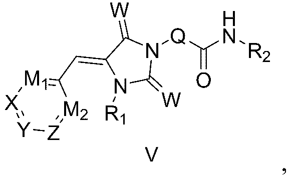 wherein W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₃, and R₃ being hydrogen, alkyl, cyano, halogen, haloalkyl, hydroxy, mercapto, alkoxy, alkylthio, alkoxyalkyl, arylalkyl, aryl or Het;
Q is selected from the group consisting of a saturated or unsaturated straight or branched hydrocarbon group having a length of 1 to 9 carbon atoms, aryl and Het;
R₁ is hydrogen, deuterium, alkyl, haloalkyl, hydroxy, mercapto, alkoxy, alkylamino, alkylthio, alkoxyalkyl, methylene, arylalkyl, diarylalkyl, aryl or Het;
R₂ is selected from the group consisting of hydroxy, and 2-aminophenyl optionally substituted by one or more R₄;
R₄ is hydrogen, alkyl, cyano, halogen, haloalkyl, hydroxy, mercapto, alkoxy, alkylthio, alkoxyalkyl, arylalkyl, aryl or Het;
the alkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 6 carbon atoms; the alkoxy is a straight or branched saturated hydrocarbon group having 1 to 9 carbon atoms, or a cyclic saturated hydrocarbon group having 3 to 9 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an oxygen atom;
the alkylamino is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated hydrocarbon group having 1 to 9 carbon atoms, wherein each carbon atom is optionally substituted by an NH atom;
the alkoxyalkyl represents linking the alkoxy defined as above to alkyl;
alkenyl or alkynyl is a straight or branched unsaturated hydrocarbon group having 1 to 9 carbon atoms and a double or triple bond;
the aryl is a carboatomic ring selected from the group consisting of phenyl, naphthyl, acenaphthenyl and tetrahydronaphthyl, each of which is optionally substituted by 1, 2 or 3 substituents, with each substituent independently selected from the group consisting of hydrogen, alkyl, cyano, halogen, haloalkyl, hydroxy, mercapto, alkoxy, alkylthio, alkoxyalkyl, arylalkyl, diarylalkyl, aryl and Het;
the arylalkyl or diarylalkyl represents linking the aryl defined as above to alkyl;
the Het is a monoheterocyclic ring selected from the group consisting of pyrrolyl, pyrazolyl, imidazolyl, furyl, thienyl, oxazolyl, isoxazolyl, thiazolyl, isothiazolyl, pyridinyl, pyrimidinyl, pyrazinyl and pyridazinyl, or is a biheterocyclic ring selected from the group consisting of quinolyl, quinoxalinyl, indolyl, benzoimidazolyl, benzooxazolyl, benzoisoxazolyl, benzothiazolyl, benzoisothiazolyl, benzofuryl, benzothienyl, 2,3-dihydrobenzo[1,4]dioxin and benzo[1,3]dioxol, or is selected from the group consisting of a monocyclic saturated hydrocarbon group having 3 to 6 carbon atoms, and a bicyclic saturated hydrocarbon group having 6 to 12 carbon atoms, wherein a carbon atom on a ring is independently and optionally substituted by 1 to 4 O, S, N or NH; each monocyclic or bicyclic ring is optionally substituted by 1, 2 or 3 substituents, with each substituent independently selected from the group consisting of halogen, haloalkyl, hydroxy, alkyl and alkoxy; the halogen is a substituent selected from the group consisting of fluorine, chlorine, bromine and iodine; and the haloalkyl is a straight or branched saturated hydrocarbon group having 1 to 9 carbon atoms, or a cyclic saturated hydrocarbon group having 3 to 6 carbon atoms, or a cyclic saturated hydrocarbon group having 3 to 6 carbon atoms and linked to a straight or branched saturated alkyl group having 1 to 6 carbon atoms, wherein one or more carbon atoms are substituted by one or more halogen atoms.

2. The imidazolidinedione-based HDAC inhibitor according to claim 1, wherein
in the Formula V, W is selected from the group consisting of an oxygen atom and a sulfur atom; X, Y, Z, M₁ and M₂ each independently represent a carbon or nitrogen atom, when X, Y, Z, M₁ or M₂ is a carbon atom, each is independently and optionally substituted by R₃, and R₃ is hydrogen, alkyl, cyano, halogen, haloalkyl, alkoxy, alkylthio, or alkoxyalkyl;
Q is selected from the group consisting of a saturated or unsaturated straight or branched hydrocarbon group having a length of 1 to 9 carbon atoms, and aryl;
R₂ is selected from the group consisting of hydroxy, and 2-aminophenyl optionally substituted by one or more R₄; and
R₄ is hydrogen, alkyl, cyano, halogen, haloalkyl, aryl or Het.

3. The imidazolidinedione-based HDAC inhibitor according to claim 1, wherein
in the Formula V, W is selected from the group consisting of an oxygen atom and a sulfur atom;
X, Y, Z, M₁ and M₂ each independently represent a carbon or nitrogen atom, when X, Y, Z, M₁ or M₂ is a carbon atom, each is independently and optionally substituted by R₃, and R₃ is hydrogen, alkyl, halogen or alkoxy;
Q is selected from the group consisting of a saturated or unsaturated straight or branched hydrocarbon group having a length of 3 to 9 carbon atoms, and aryl;
R₂ is selected from the group consisting of hydroxy, and 2-aminophenyl optionally substituted by one or more R₄; and
R₄ is hydrogen, alkyl, cyano, halogen, haloalkyl, aryl or Het.

4. The imidazolidinedione-based HDAC inhibitor according to claim 1, wherein in the Formula V, hydrogen attached to carbon is replaced by deuterium, which is an isotope of hydrogen.

5. The imidazolidinedione-based HDAC inhibitor according to claim 1, wherein the inhibitor is one of:
(1) (Z)-N-hydroxy-7-(4-(2-methoxybenzylene)-2,5-dioxoimidazolidin-1-yl)heptanamide;
(2) (Z)-N-hydroxy-8-(4-(2-methoxybenzylene)-2,5-dioxoimidazolidin-1-yl)octanamide;
(3) (Z)-N-hydroxy-6-(4-(2-methoxybenzylene)-2,5-dioxoimidazolidazol-1-yl)hexanamide;
(4) (Z)-N-hydroxy-5-(4-(2 -methoxybenzylene)-2,5-dioxoimidazolidin-1-yl)pentanamide;
(5) (Z)-N-hydroxy-7-(4-(4-bromo-2-methoxybenzylene)-2,5-dioxoimidazolidin-1-yl)heptanamide;
(6) (Z)-N-hydroxy-8-(4-(4-bromo-2-methoxybenzylene)-2,5-dioxoimidazolidin-1-yl)octanamide;
(7) (Z)-N-hydroxy-9-(4-(2-methoxybenzylene)-2,5-dioxoimidazolidin-1-yl)nonanamide;
(8) (Z)-N-hydroxy-10-(4-(2-methoxybenzylene)-2,5-dioxoimidazolidin-1-yl)decanamide;
(9) (Z)-N-hydroxy-7-(4-(2-methoxybenzylene)-3-methyl-2,5-dioxoimidazolidin-1-yl)heptanamide;
(10) (Z)-N-hydroxy-7-(4-(4-bromo-2-methoxybenzylene)-3-methyl-2,5-dioxoimidazolidin-1-yl)heptanamide;
(11) (Z)-N-hydroxy-6-(4-(4-bromo-2-methoxybenzylene)-2,5-dioxoimidazolidin-1-yl)hexanamide;
(12) (Z)-N-hydroxy-6-(4-(3-methoxybenzylene)-2,5-dioxoimidazolidin-1-yl)hexanamide;
(13) (Z)-N-hydroxy-6-(4-(4-methoxybenzylene)-2,5-dioxoimidazolidin-1-yl)hexanamide;
(14) (Z)-N-hydroxy-6-(4-(2,4-dimethoxybenzylene)-2,5-dioxoimidazolidin-1-yl)hexanamide;
(15) (Z)-N-hydroxy-6-(4-(4-fluoro-2-methoxybenzylene)-2,5-dioxoimidazolidin-1-yl)hexanamide;
(16) (Z)-N-hydroxy-6-(4-(4-chloro-2-methoxybenzylene)-2,5-dioxoimidazolidin-1-yl)hexanamide;
(17) (Z)-N-hydroxy-6-(4-(2-methoxy-4-methylbenzylene)-2,5-dioxoimidazolidin-1-yl)hexanamide;
(18) (Z)-N-hydroxy-6-(4-(2-methoxy-4-(trifluoromethyl)benzylene)-2,5-dioxoimidazolidin-1-yl)hexanamide;
(19) (Z)-N-hydroxy-6-(2.5-dioxo-4-(3,4,5-trimethoxybenzylene)imidazolidin-1-yl)hexanamide;
(20) (Z)-N-hydroxy-6-(4-(naphth-1-ylmethylene)-2,5-dioxoimidazolidin-1-yl)hexanamide;
(21) (Z)-N-hydroxy-6-(4-(naphth-2-ylmethylene)-2,5-dioxoimidazolidin-1-yl)hexanamide;
(22) (Z)-N-hydroxy-6-(2,5-dioxo-4-(quinolin-2-ylmethylene)imidazolidin-1-yl)hexanamide;
(23) (Z)-N-hydroxy-6-(2,5-dioxo-4-(quinolin-3-ylmethylene)imidazolidin-1-yl)hexanamide;
(24) (Z)-N-hydroxy-6-(2,5-dioxo-4-(quinolin-4-ylmethylene)imidazolidin-1-yl)hexanamide;
(25) (Z)-N-hydroxy-6-(2,5-dioxo-4-(quinolin-6-ylmethylene)imidazolidin-1-yl)hexanamide;
(26) (Z)-N-hydroxy-6-(2,5-dioxo-4-(quinolin-5-ylmethylene)imidazolidin-1-yl)hexanamide;
(27) (Z)-N-hydroxy-6-(4-(furan-2-ylmethylene)-2,5-dioxoimidazolidin-1-yl)hexanamide;
(28) (Z)-N-hydroxy-6-(2,5-dioxo-4-(thiophen-2-ylmethylene)imidazolidin-1-yl)hexanamide;
(29) (Z)-N-hydroxy-6-(4-((2,3-dihydrobenzofuran-5-yl)methylene)-2,5-dioxoimidazolidin-1-yl)hexanamide;
(30) (Z)-N-hydroxy-6-(4-(benzofuran-5-ylmethylene)-2,5-dioxoimidazolidin-1-yl)hexanamide;
(31) (Z)-N-hydroxy-6-(4-(benzo[d][l,3]dioxol-5-ylmethylene)-2,5-dioxoimidazolidin-1-yl)hexanamide;
(32) (Z)-N-hydroxy-6-(4-((2,2-difluorobenzo[d][1,3]dioxol-5-yl)methylene)-2,5-dioxoimidazolidin-1-yl)hexanamide;
(33) (Z)-N-(2-amino-4-fluorophenyl)-4-((2,5-dioxo-4-(pyridin-3-ylmethylene)imidazolidin-1-yl)methyl)benzamide;
(34) (Z)-N-(2-amino-4-fluorophenyl)-4-((2,5-dioxo-4-(pyridin-3-ylmethylene)imidazolidin-1-yl)methyl)benzamide; and
(35) (Z)-N-hydroxy-6-(2,5-dioxo-4-((4-(pyridin-4-yl)quinolin-6-yl)methylene)imidazolidin-1-yl)hexanamide.

6. A method for preparing the imidazolidinedione-based HDAC inhibitor according to claim 5, comprising: subjecting substituted formaldehyde (A) and hydantoin or substituted hydantoin (B), as starting materials, to a Knoevenagel condensation reaction under the action of alanine to obtain an intermediate (C); subjecting the intermediate (C) and bromocarboxylate to a nucleophilic substitution reaction to obtain a key intermediate (D); hydrolyzing the intermediate (D) under acidic conditions to obtain an intermediate (E); and condensing the intermediate (E) with hydroxylamine hydrochloride to obtain a target compound (1-32), with a synthesis route as below:

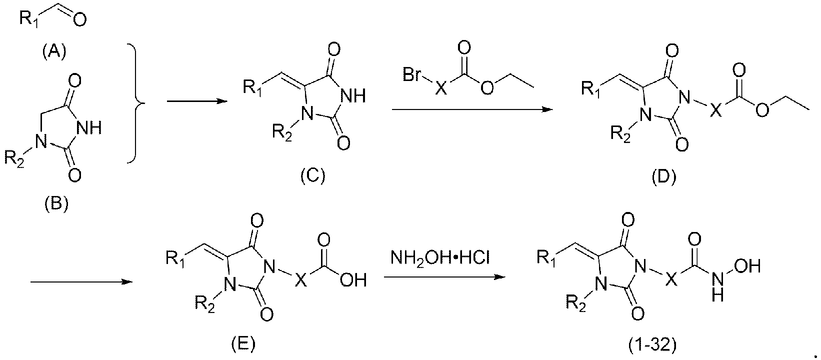

7. A method for preparing the imidazolidinedione-based HDAC inhibitor according to claim 5, comprising: subjecting substituted formaldehyde (A) and hydantoin or substituted hydantoin (B), as starting materials, to a Knoevenagel condensation reaction under the action of alanine to obtain an intermediate (C); subjecting the intermediate (C) and bromocarboxylate to a nucleophilic substitution reaction to obtain a key intermediate (D); hydrolyzing the intermediate (D) under acidic conditions to obtain an intermediate (E); and condensing the intermediate (E) with 4-fluoro-1,2-phenylenediamine to obtain a target compound (33-34), with a synthesis route as below:

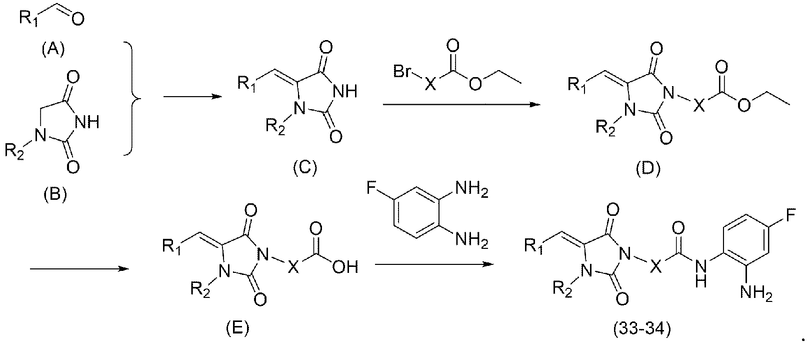

8. A method for preparing the imidazolidinedione-based HDAC inhibitor according to claim 5, comprising: subjecting a compound (F), as a starting material, and cycl-isopropylidene malonate to a Combes quinoline synthesis reaction to obtain an intermediate (G); subjecting the intermediate (G) and phosphorus tribromide to a bromination reaction in DMF to obtain an intermediate (H); subjecting the intermediate (H) and 4-pyridineboronic acid to a Suzuki coupling reaction to obtain an intermediate (I); and subjecting the intermediate (I) and lithium aluminum hydride to a reduction reaction to obtain an intermediate (J); and subjecting the intermediate (J) to selective oxidation to obtain a key intermediate (K), and subjecting the intermediate (K) to four sequential reactions to obtain a target compound (35), with a synthesis route as below:

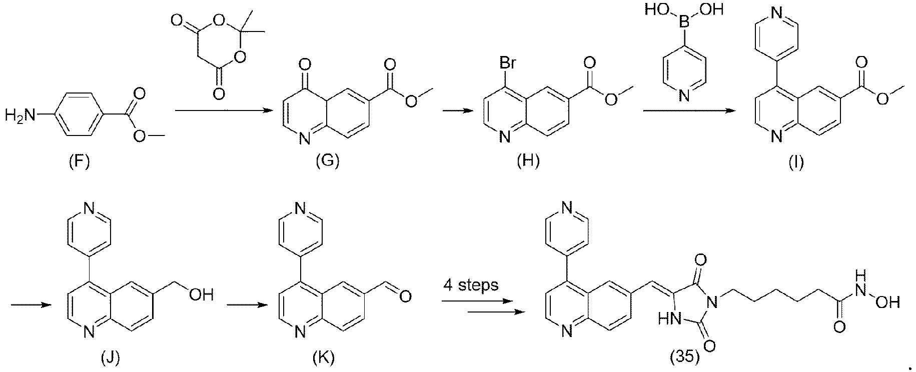

9. A pharmaceutical composition, comprising the inhibitor according to any one of claims 1 to 5 or a pharmaceutically acceptable salt thereof, and a pharmaceutically acceptable carrier, wherein the inhibitor according to any one of claims 1 to 5 or the pharmaceutically acceptable salt thereof is an active ingredient.

10. Use of the inhibitor according to any one of claims 1 to 5 in the preparation of a medicament for preventing or treating HDAC-associated clinical disorders.

11. The use of the inhibitor according to claim 10, wherein the HDAC-associated clinical disorders comprise lung cancer, melanoma, liver cancer, kidney cancer, leukemia, prostate cancer, thyroid cancer, skin diseases, pancreatic cancer, ovarian cancer, testicular cancer, breast cancer, bladder cancer, gallbladder cancer, myelodysplastic syndrome, lymphoma, esophageal cancer, gastrointestinal cancer, astrocytoma, neuroblastoma, glioma, neurilemoma, mesothelioma, non-insulin-dependent diabetes mellitus, and autoimmune diseases.
